# EUROPEAN PATENT SPECIFICATION

(11) **EP 3 161 481 B1**
(45) Date of publication and mention of the grant of the patent: **16.10.2024**
(21) Application number: 15812274.7
(22) Date of filing: 26.06.2015
(51) Int. Cl.: G16B 20/00, G16H 50/20, G16H 50/30, G01N 33/50, G01N 33/569, G16B 20/20

(54) **SYSTEM FOR ASSESSING GLOBAL WELLNESS**
SYSTEM ZUR BEURTEILUNG VON GLOBALEM WOHLBEFINDEN
SYSTÈME D'ÉVALUATION DU BIEN-ÊTRE GLOBAL

(30) Priority: 28.06.2014 US 201462018542 P
(43) Date of publication of application: 03.05.2017
(73) Proprietor: Relevance Health, Frisco, Texas 75034 (US); Wade, Jill, Frisco, Texas 75034 (US)
(72) Inventor: WADE, Jill, Frisco, Texas 75034 (US)
(74) Representative: Sagittarius IP
(86) International application number: PCT/US2015/038183
(87) International publication number: WO 2015/200898

(56) References cited:
- US-A1- 2008 162 352
- US-A1- 2008 162 352
- US-A1- 2011 093 249

## Description

### TECHNICAL FIELD

The present disclosure relates generally to the fields of dentistry, medicine, physiology, diagnostics, and biochemistry. More specifically, the subject matter described herein relates to systems and methods for assessing physiological indicators and biological markers to provide an assessment of global wellness of a subject and subsequently or concurrently developing a maintenance and/or treatment program.

### BACKGROUND

Stress is a leading cause of inflammation, which itself underlies chronic diseases such as cardiovascular disease (CVD), gingivitis or periodontal disease, diabetes, autoimmune diseases, and cancers, for example. "Address the Stress" (Dog Ear Publishing, LLC. October 14, 2011, by Drs. Jill Wade and Kelly Martin) is a guide for people dealing with the health manifestations of stress.

To date, much is known about diseases related to inflammation and their impact on health. For example, diabetes mellitus is a heterogeneous group of metabolic diseases characterized by elevated blood glucose levels and increased morbidity. Both genetic and environmental factors contribute to its development. The most common form is T2D, which is characterized by defects in both insulin secretion and insulin action. In contrast, type I diabetes results from autoimmune destruction of the insulin-producing beta cells of the pancreas. Monogenic forms of diabetes account for less than 5% of the cases and are usually caused by mutations in genes associated with maturity-onset diabetes of the young (MODY), insulin gene and insulin receptor gene. T2D is a heterogeneous disease resulting from the interaction of environmental factors such as obesity or sedentary lifestyle, with variety of diabetogenic genes. Abnormal glucose homeostasis occurs when either insulin sensitivity or insulin secretion or both are altered, and an early sign of its development is insulin resistance, defined as impaired insulin-mediated glucose clearance in insulin-sensitive tissues (skeletal muscle, liver and adipose tissue). Elevation of glucose levels triggers beta-cells to produce and secrete more insulin, which compensates for the disturbance in glucose homeostasis. The duration of hyperglycemia-hyperinsulinemia state depends on insulin secretory capacity, mass and apoptosis rate of beta-cells. Furthermore, beta-cells can lose their insulin secretion capacity because of glucose toxicity or other reasons. When cells fail to compensate for insulin resistance blood glucose concentration increases. Thus, over time subclinical hyperglycemia tends to progress to impaired glucose tolerance and further to T2D.

No major single gene explaining the development of T2D has been identified. There were several studies attempting to predict T2D based on limited number of SNPs (Lyssenko, et al., Genetic prediction of future type 2 diabetes. PLoS Med 2, e345 (2005); Meigs, et al., Genotype score in addition to common risk factors for prediction of type 2 diabetes. N Engl J Med 359, 2208-19 (2008); Cauchi, et al., Post genome-wide association studies of novel genes associated with type 2 diabetes show gene-gene interaction and high predictive value. PLoS ONE 3, e2 3 1 (2008); Miyake, et al., Construction of a prediction model for type 2 diabetes mellitus in the Japanese population based on 1 1 genes with strong evidence of the association. J Hum Genet 54, 236-41 (2009); Lin, et al., Risk prediction of prevalent diabetes in a Swiss population using a weighted genetic scorethe CoLaus Study. Diabetologia 52, 600-8 (2009)). However, with all of these methods, the predictive power was found to be limited in practice, and better models are needed.

Cardiovascular disease (CVD) is also an important health concern that has enormous social and economic consequences. CVD is the leading cause of death in developed countries: in the United States alone, the projected cost of CVD in 2005 was estimated at $431.8 billion, including health care services, medications, and lost productivity. Atherosclerotic Heart Disease (ASHD) or coronary artery disease (CAD), a cardiovascular disease condition, develops when lipids and inflammatory cells accumulate in the walls of coronary arteries, forming atherosclerotic plaques. As CAD progresses, clinical manifestations may develop, including the occurrence of angina. Acute Coronary Syndrome (ACS), which includes unstable angina and acute myocardial infarction (AMI), is associated with plaque rupture and thrombus formation in a coronary vessel, resulting in myocardial ischemia and often necrosis. According to the American Heart Association (Heart and Disease Statistics - 2004), the following dire morbidity and mortality statistics were associated with CAD in the United States: CAD is the primary cause of death in America today and was responsible for more than one third of U.S. deaths in 2004. Further, 13.2 million people (7.2 million males and 6.0 million females) living today have experienced a heart attack, angina or both, approximately 330,000 people a year will die of an ACS event inside or outside of the emergency room and 1.2 million Americans are expected to have a new or recurrent coronary event this year. In 2008, an estimated 770,000 Americans will have a new coronary attack, and about 430,000 will have a recurrent attack. It is estimated that an additional 175,000 silent first myocardial infarctions occur each year. Here, about every 26 seconds, an American will have a coronary event, and about every minute someone will die from a coronary event

Unfortunately, at present, scientific and medical communities are faced with disjointed information based on non-standardized data and multiple disparate test results achieved on separate instruments. Understanding the complex pathobiology of the wide variety of cardiovascular diseases, diabetes, and other human health concerns, and the ability to apply that knowledge to assess the risk and timing of future health or disease events will help develop improve diagnostic tests, and prevent or minimize some of the adverse outcomes of disease. There is a need for additional methods for biomarker identification and validation, as well as methods for diagnosing and prognosing a broad range of diseases pertinent to maintaining wellness.

Wellness programs have been in vogue for the past several years. In some cases, systems have been described which to generate a wellness program for a user, in which personal data items are entered into a computing system corresponding to a personal profile of the user. However, none described to date uses a global approach and using systems and methods for assessing physiological indicators, biological markers (pathogenic, genetic and protein), and the tools of dentistry, medicine, physiology, diagnostics, and biochemistry all together, to meet the long-felt need to address the root source(s) of disease, and identify and address/reduce/remove the stressors of life by providing an assessment of global wellness status of a subject and subsequently or concurrently designing a maintenance and/or treatment program to achieve an ideal state of global wellness.

US 2008/162352 A1 discloses a system for assessing global wellness and developing a treatment plan for a subject in general. However, this document fails to disclose the conception of an oral-systemic link between oral health or disease and whole-body wellness.

### BRIEF SUMMARY

The invention is defined by the appended claims.

The present disclosure bridges a gap between standard medical and dental practices. In one aspect, the disclosure provides a system for assessing global wellness and developing a maintenance / treatment plan for a subject, comprising
(i) a computer-readable storage medium comprising a recorded medical and dental history of the subject;
(ii) a sample-collection apparatus for obtaining an oral sample from the subject, comprising a plurality of analyte detection regions in direct or indirect fluid communication with said sample-collection apparatus, wherein the analyte detection regions test for the presence of
   (a) nucleic acids from bacterial or pathogens; and
   (b) endogenous genetic biomarkers of inflammation and infection;
   and wherein the analyte detection regions further provide a measure of levels of specific hormones or lipids; and
(iii) a computer processor for assessing data from the analyte detection regions of (ii) and the recorded medical and dental history of (i), wherein the processor computes and provides an output recommendation for a maintenance / treatment program.

In another aspect, the present disclosure provides a method for assessing global wellness and developing a maintenance / treatment plan for a subject, comprising:
(i) compiling medical and dental history data points from a subject into a system comprising a computer-readable form;
(ii) obtaining an initial periodontal status from a biological sample from the subject;
(iii) comparing the medical and dental history data points and the initial periodontal status to a reference set of desired health and wellness goals;
(iv) comparing the periodontal status, medical and dental history data points to the reference set of desired restored health and wellness goals;
(v) computing a path / series of behavioral steps toward restored health and wellness from the initial periodontal status, medical and dental history data points, through optional intermediate stepped results, to the reference set of desired health and wellness goals according to a minimum step criterion; and
(vi) determining a treatment plan to adjust the subject's behaviors from the initial periodontal status, medical and dental history data points to the reference set of desired health and wellness goals.

In some embodiments, the method further comprises persistently storing the subject's actual achieved intermediate stepped results as a function of time. In some embodiments, the method further comprises re-computing a path (a series of behavioral steps) toward restored health and wellness, and in some embodiments, altering the treatment plan based on the actual achieved intermediate stepped results as a function of time.

In addition to the exemplary aspects and embodiments described above, further aspects and embodiments will become apparent by reference to the drawings and by study of the following descriptions.

### BRIEF DESCRIPTION OF DRAWINGS

Figure 1: shows the Circle of Health, including parameters of global wellness;
Figure 2: shows the oral-systemic link, including the connections between oral health (or disease) and whole-body wellness (or disease);

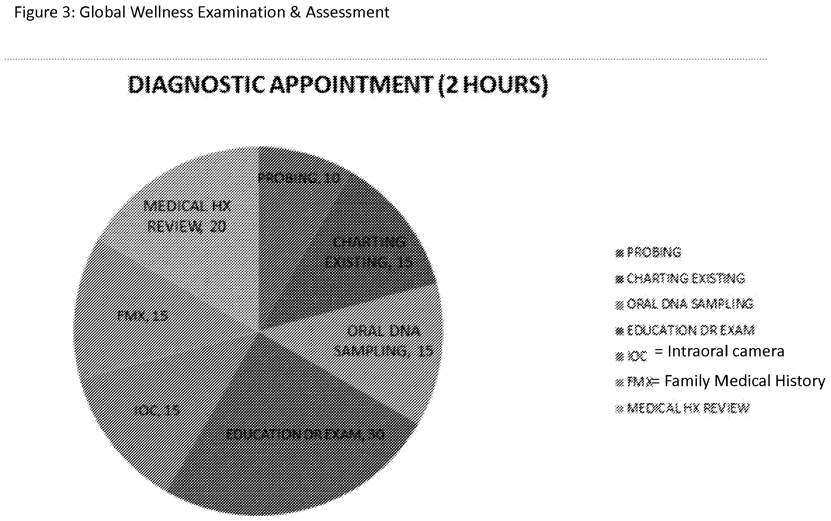

Figure 3: graphically presents an exemplary Examination & Assessment of Global Wellness;
Figure 4: exemplifies one examination form used to assess Global Wellness;
Figure 5: maps out a scheme for the Smile With Heart (SWH) process;
Figure 6: maps out the data collection, assessment and treatment planning process in a method and system for achieving Global Wellness; and
Figure 7 shows a time line representing a subject's medical and dental history and a prescribed path to wellness.

### DETAILED DESCRIPTION

### I. Definitions

As used throughout the present disclosure, the technical and scientific terms used in the descriptions herein will have the meanings commonly understood by one of ordinary skill in the art, unless specifically defined otherwise. Accordingly, the following terms are intended to have the following meanings:

As used in this specification and the appended claims, the singular forms "a," "an," and "the" include plural referents unless the context clearly indicates otherwise. Thus, for example, reference to a subject's "gene" can include more than one gene, reference to a "device" includes a single device as well as two or more of the same or different devices, and reference to a "test" refers to a single test as well as two or more tests. The use of "or" should be understood to mean "and/or" unless stated otherwise. Similarly, "comprise," "comprises," "comprising" "include," "includes," "including," "has," "have" and "having" are interchangeable and not intended to be limiting. It is also to be understood that where descriptions of various embodiments use the term "comprising," those skilled in the art would understand that in some specific instances, an embodiment can be alternatively described using language "consisting essentially of" or "consisting of."

The present disclosure bridges a gap between standard medical and dental practices, and relates generally to the fields of dentistry, medicine, physiology, diagnostics, and biochemistry. More specifically, the subject matter described herein relates to systems and methods for assessing physiological indicators and biological markers to provide an assessment of global wellness of a subject and subsequently or concurrently developing a maintenance and/or treatment program. More particularly, the disclosure is directed to a system in which a plurality of data sources are used to generate a landscape of particular data points representing an individual's initial health and wellness status, which landscape of particular data points is then used, with optional assistance by a computer program, to map a therapeutic course to change the individual's initial health and wellness state to a desired state of wellness and provide a treatment plan for the subject.

Periodontal status provides a window into the body's systemic health. The oral environment can provide many subtle hints to the processes of aging, stress level, and disease (not only oral disease, but the entire body's health and/or predisposition to diseases such as diabetes, cardiovascular disease, stroke, etc. Furthermore, the mouth is an indicator of nutritional status, and provides a means to modify the oral as well as global systemic environment of the body. It is increasingly clear that health of the oral cavity is strongly linked to our systemic body as a whole - the oral systemic link - and a dental as well as medical history can provide critical data for diagnosing global health and wellness and providing a strategy and treatment plan to achieving improved health and wellness. Overall, the present disclosure provides a means of systematically approaching and organizing the assessment of patients (subjects) seeking to improve their global health and wellness.

Chronic inflammatory disease is one key to the oral systemic link. The same inflammatory response that occurs in the mouth as gingivitis is the same type of inflammatory response that can be occurring in the arteries having a negative effect on the heart. Heart disease is the number one cause of death. About every 25 seconds, an American will have a coronary event. Every one minute someone will die from a heart attack.

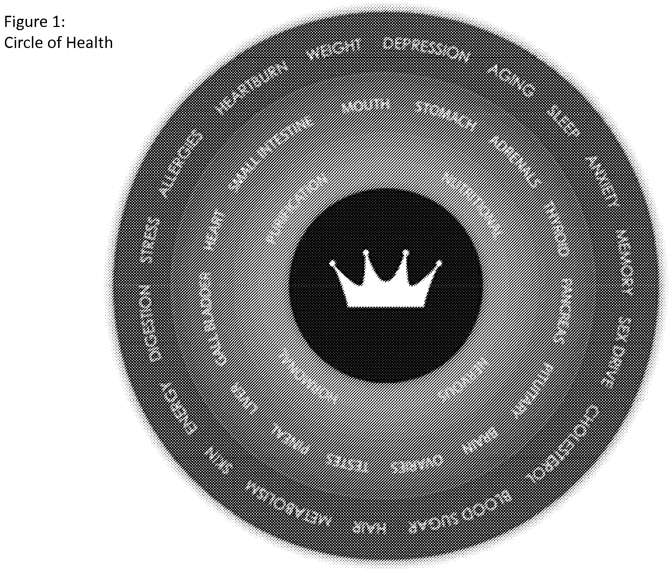

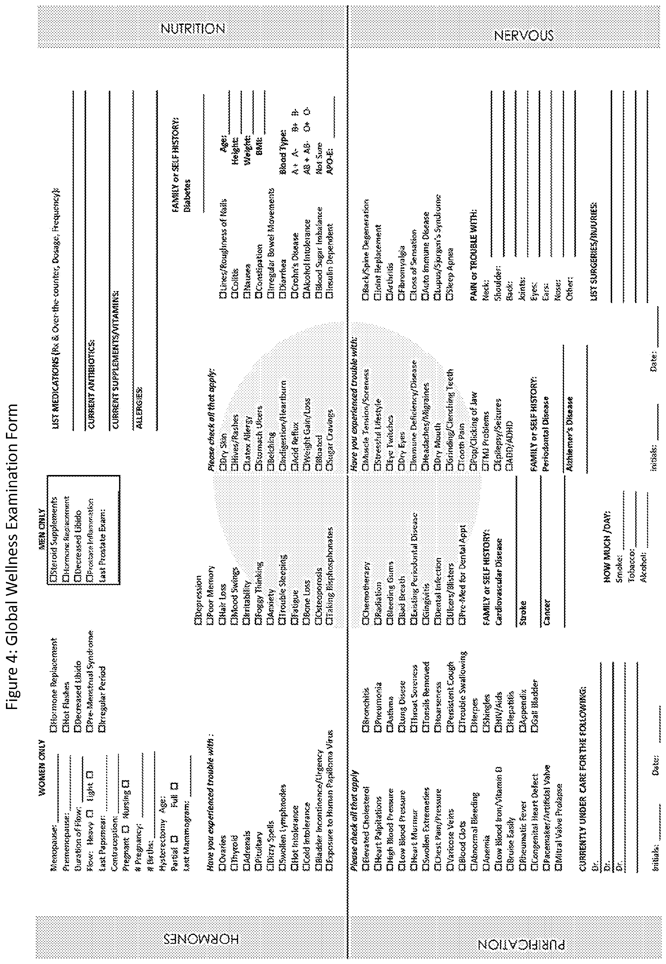

The "Circle of Health" (see Figure 1) is a philosophical approach used to help understand the comprehensive nature of the oral-systemic link and to arrive at a conception of global wellness. The body is treated as a whole rather than as disconnected parts or isolated systems. The "Circle of Health" has four core elements: nutrition, hormones, nervous, and purification (which are also shown as four sectors of a circle shown in the middle of Figure 4); the Circle of Health also includes three rings: the outer ring of symptoms, a middle ring of organ systems, and an inside ring containing four core elements (a.k.a. "crown jewels) which are hormones, nutrition, purification, and neurons/nervous system. The present disclosure addresses a long-felt need for a system and method involving testing, treating, and achieving results in preventing cardiovascular diseases alongside periodontal disease; it cleverly unites state-of-the-art approaches in both dentistry and medicine via a unique simultaneous assessment of cardiovascular, inflammatory and dental indicators. The system and method of global wellness assessment herein disclosed incorporates both dental and medical histories, a subject's family history and genetics, careful observations of clinical signs, patient self-reporting and periodontal and oral DNA testing for pathogens, oral DNA testing of genetic markers of risk factors, results from test strips for testing pH, for example, (as well as other parameters) in saliva or urine, medical blood work or advanced lipid testing, Carotid Intima-Media Thickness (CIMT) ultrasound, inflammatory markers, and other genetic tests, and clinical exam of physician/cardiologist, which data-gathering results in a global landscape for an oral-systemic healthcare provider and patient to find suggestions / prescriptions and guidance toward a healthier overall state of wellness.

An oral-systemic link is well established, but, to date, no system has been developed to assess medical and dental aspects of chronic Inflammation together, to develop a global intervention/ treatment/prevention plan. The presently disclosed comprehensive approach to wellness does not divide the body into part and pieces, but remains cognizant that the body functions as a whole. Chronic inflammation is the key to the metabolic diseases our society is facing. Periodontal disease is the #1 global disease. Cardiovascular disease is the number 1 killer effecting hundreds of thousands of deaths per year. Chronic inflammation stimulating free radicals and then oxidative stress catalyze metabolic diseases that lead to CVD, diabetes, metabolic syndrome, insulin resistance, cancer, premature aging, autoimmune diseases, strokes, periodontal disease, etc. Even natural aging and imbalances in hormones, stress, chronic inflammation, acid reflux, sleep disorders, nutritional deficiencies, early signs of diabetes, and cancers can be seen in the oral cavity.

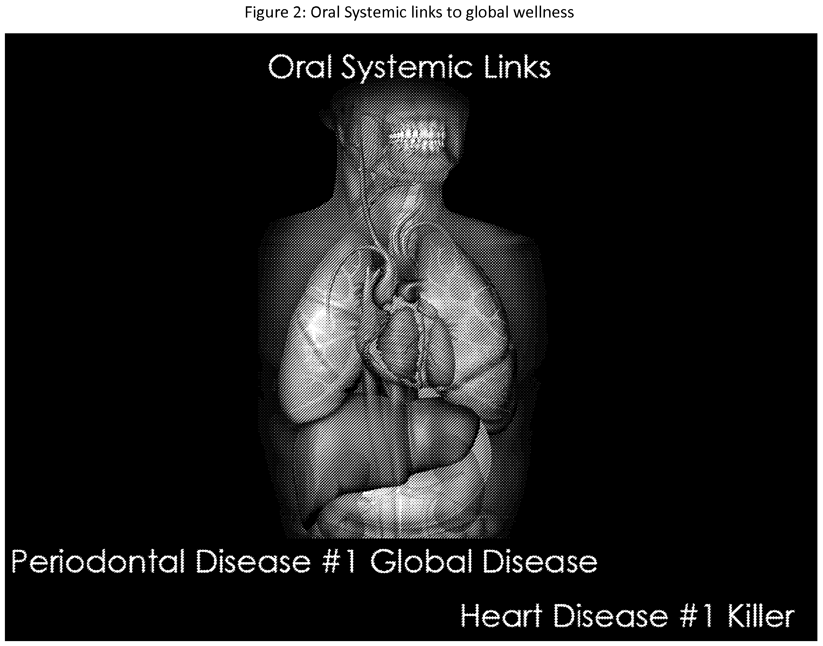

As the start of the digestive system, the oral cavity plays a critical role in wellness; oral environment is affected by pH from prescriptions, dry mouth, diabetes, food ingested, physical oral care, and is one of the only direct accesses of bacteria into the blood stream via the vascular area directly around the teeth. While a few other entry zones exist, such as open wounds and the vasculature of digestive / gastrointestinal tract, the importance of the oral-systemic link (see Figure 2) is a crucial consideration to overall health and wellness, and has remained underdiagnosed. Nonetheless, it is clear that the burden on the body from early through late stage periodontitis, and/or undiagnosed dental abscesses prevent systemic health from ever being optimal.

This simultaneous, complimentary (and potentially synergistic) approach of medical and dental assessment of global wellness and development of targeted interventions to decrease inflammation can achieve far better results for a subject's global health and wellness than has been achieved with medicine and dentistry as separate practices.

Continued re-testing after certain time intervals allows all participants to observe and understand the subject's individual response to the prescribed intervention / treatment / maintenance therapy and preventive care. Interventions can include nutrition, changes from a sedentary to a more active lifestyle, weight loss, quitting smoking, quitting drinking alcohol, and even replacing silver-mercury fillings can all have an impact on one's global health and wellness; these can benefit most individuals, but using the systems and methods disclosed herein to design particular programs of intervention targeted to particular states of illness or predisposition to illness, one can arrive at even better, more ideal states of health and wellness.

As used herein, the phrase "adverse cardiovascular outcome" associated with or resulting from atherosclerosis refers to coronary artery disease (CAD), ischemic heart disease (IHD), angina pectoris, AMI, death, stroke or peripheral vascular disease. The phrase "coronary artery disease" refers to atherosclerotic disease of the coronary arteries, but also infers probable atherosclerotic disease of the peripheral arteries such as those supplying the legs and the brain, and includes the consequences of CAD, such as myocardial infarction and death, angina pectoris, stroke and peripheral vascular disease.

The term "biomarker" refers to a distinctive biological or biologically derived indicator of a process, event, or condition. Biomarkers as used herein encompass, without limitation, gene products, including proteins, nucleic acids, and metabolites, together with their polymorphisms, mutations, variants, modifications, subunits, fragments, protein-ligand complexes, and degradation products, protein-ligand complexes, elements, related metabolites, and other analytes or sample-derived measures that are associated with a biological state.

Biomarkers can also include mutated proteins or mutated nucleic acids. Biomarkers preferably include inflammatory, infection, thrombotic or autoimmune biomarkers.

The phrase "inflammation biomarker" or "biomarker of inflammation," as used herein, refers to a biomarker that is an indicator of inflammation. Exemplary inflammation biomarkers include C-reactive protein (CRP), interleukin 1 composite genotype, interleukin (IL)-6, interleukin 17A, beta-defensin 1, CD 14, tumor necrosis factor alpha, toll-like receptor 4 composite genotype, matrix metalloproteinase 3, and serum amyloid A protein, homocysteine.

The phrase "biomarker of infection" or "infection biomarker," as used herein, refers to a biomarker that is an indicator of infectious diseases. A list of potential infection biomarkers (biomarkers of infection) can be found at the Infectious Disease Biomarker Database (IDBD) (See biomarker.cdc.go.kr and biomarker.korea.ac.kr). Exemplary infection biomarkers include gene products of (including proteins): CRP, anti-cytomegalovirus (CMV) antibody, Chlamydia pneumonia, herpes simplex virus (HSV) types 1 and 2, Helicobacter pylori, and hepatitis A virus, as well as periodontal pathogens.

"Autoimmune disease biomarker" or "biomarker of autoimmune disease," as used herein, refers to a biomarker that is an indicator of autoimmune disease. Three groups of gene products, e.g., proteins, are reflective of the autoimmune disease process: (1) degradation products arising from destruction of affected tissues, (2) enzymes that play a role in tissue degradation and (3) cytokines and other proteins associated with immune activation (Prince, H.E., Biomarkers, 2005, Nov. 10, Suppl 1: S44-49). Exemplary autoimmune disease biomarkers include gene products of (including proteins): antibody to Heat Shock Protein 60 (anti-HSP60), Heat Shock Protein 70 (HSP70), aggrecan fragments, C-propeptide of type II collagen and cartilage oligomeric matrix protein, matrix metalloprotease (MMP)-I, MMP-3 and MMP- 1/inhibitor complexes thioredoxin, IL-16 and tumour necrosis factor (TNF)-alpha, neurofilament light protein and glial fibrillary acidic protein, MMP-2 and MMP-9 and TNF-alpha and soluble vascular adhesion molecule-1.

"Cellular stress biomarker," or "biomarker of cellular stress," as used herein, refers to a biomarker, the levels of which increase when a cell is exposed to stress. Exemplary cellular stress biomarkers include Heat Shock Protein (HSP) 70 (HSP70), and certain other HSPs, such as HSP32, HSP27, HSP72, HSP90, HSP47, as well as ubiquitin, and Hsc70, and cellular stress biomarkers discussed by Rajdev and Sharp, Toxicologic Pathology, 28(1) 105-112 (2000); and Zhou et al, Circulation, 110: 207-213 (2004).

The phrase "biomarker of thrombosis," or "thrombosis biomarker," as used herein, refers to a biomarker that is an indicator of thrombosis. Some examples include fibrinogen, prothrombin 1.2, tissue plasminogen activator antigen (tPA), plasminogen activator inhibitor- 1 (PAI-1), and FDP markers, such as an FDP marker that includes a mixture of at least two fibrin and fibrinogen degredation products (FDPs), such as two or more of fragments X, Y, D, D- dimer, and E fragment Y, and initial plasmin digest products (IPDP).

As used herein, the phrases/terms "fibrin degradation product(s)," "fibrin and fibrinogen degradation product(s)," and "FDP" refer to one or more fragments produced when either fibrin or fibrinogen is degraded. FDPs include four principal FDPs, called X, Y, D, and E fragments (fragment X, fragment Y, fragment D, and fragment E) that are liberated in various combinations. Cleavage of fibrinogen by plasmin produces fragments D and E as the primary end-products. Thrombin converts fibrinogen to fibrin. When a fibrin clot is broken down by plasmin, the last fragment to be degraded (containing two D and one E subunits) is split, releasing the E fragment and also two D fragments covalently linked together (called "D- dimer"). This D-dimer is produced from fibrin, not fibrinogen degradation. Accordingly, the FDP biomarker can include the presence of one or more of X, D, and E fragments. In one example, the FDP biomarker includes fragment Y; in one example, it includes one or two distinguishable forms of initial plasmin digest product (IPDP). In some embodiment, the provided methods and systems detect a the level of a FDP marker; in one aspect, this FDP marker includes a mixture of at least two FDPs, such as fragments D, E, and D-dimer, and in some aspects further including one or more of fragments X, Y, and IPDP. In one example, the FDP marker includes one, more, or all FDPs detected by the DR-70 ELISA assay. In another example, the FDPs include one or more FDPs described in International Application Publication Number WO 2010/114514 A1. In another example they include one or more FDPs detected by a Fibrinogen ELISA assay using either anti-Fibrinogen polyclonal or multiple monoclonal antibodies.

"C-reactive protein" is also known as "hsCRP", or "CRP," and is a marker of the reactant plasma protein component of the inflammatory response. CRP is a protein produced by hepatocytes as part of the non-specific acute phase response to inflammatory conditions. It is used to diagnose and monitor a wide variety of infectious diseases.

"Heat shock protein 70" is also known as"HSP70" "HSP73" "HSPA8" Other family members include HSP 70-1, HSP 70-2, HSP 70-4, HSP 70-4L, HSP 70-5, HSP 70-6, HSP 70-7, HSP 70-8, HSP 70-9, HSP 70- 12a, HSP 70-14. Increased levels are found during conditions in which cells are exposed to stress. Thus, HSP70 is among the cellular stress biomarkers.

"Cytomegalovirus" "CMV" is a part of the herpes family of viruses. Other family members include herpes simplex virus type 1 (HSV-1 or HHV-1) and herpes simplex virus type 2 (HSV-2 or HHV-2), varicella zoster virus (VZV), human herpesvirus (HHV)-6, HHV-7, and HHV-8. The biomarker detected for CMV includes CMV antibody (CMV-Ab).

Exemplary pathogens associated with disease and identified in oral DNA testing include (but are not limited to): *Aggregatibacter actinomycetemcomitans, Campylobacter rectus, Capnocytophaga* species (*gingivalis, ochracea, sputigena*), *Eikenella corrodens, Eubacterium nodatum, Fusobacterium nucleatum* / *periodonticum, Peptostreptococcus (Micromonas) micros, Porphyromonas gingivalis, Prevotella intermedia, Tannerella forsythia, Treponema denticola,* and combinations thereof. Results from assays monitoring for these pathogens can be used in the presently described method and system in order to predict the risk / probability of occurrence of a periodontal and/or peri-implant disease in a subject.

The term "mammal" or "patient" or "subject" refers to such organisms as mice, rats, rabbits, goats, horse, sheep, cattle, cats, dogs, pigs, preferably domesticated animals such as pets, more preferably monkeys and apes, and most preferably humans. In some embodiments, the subject is a human, and the test or biological sample, which can be a test sample or a control sample, used is a bodily fluid or bodily tissue.

The terms "bodily fluids" or "body fluids" as used herein include circulating and non-circulating fluids. Examples of circulating fluids include blood, serum, CSF, and lymph fluid. Examples of non-circulating fluids include synovial fluid. Body fluids can include amniotic fluid, aqueous humour, vitreous humour, breast milk, cerebrospinal fluid (CSF), cerumen (earwax), chyle, chime, endolymph, perilymph, feces, gastric acid, gastric juice, lymph, mucus, nasal drainage, phlegm, pericardial fluid, peritoneal fluid, pleural fluid, pus, rheum, saliva, sebum, semen, serum, sputum, sweat, synovial fluid, tears, vomit, urine or exhaled condensate.

As used herein, a "digital wellness landscape" refers to digitized information about the medical and dental data points representing all that is known about a subject. A digital wellness landscape is obtained, for example, using a computer and appropriate software, data entry from medical and dental histories, oral sampling and imaging as well as whole-body imaging techniques, including but not limited to magnetic resonance imaging, laser scanning, ultrasound, x-ray, etc. The digital wellness landscape described herein can be stored in computer-readable form. The digital wellness landscape is generally capable of being represented visually and/or graphically on a computer screen or video monitor.

"Display" refers to a computer screen, video monitor, or other device capable of presenting an image to a viewer. "Display is capable of being manipulated" means that the image can be adjusted, elements added or moved on the screen or monitor to simulate, predict the effects of, or prescribe various adjustments to image.

The term "computer-readable medium" as used herein refers to any medium that participates in providing instructions to a processor for execution. Such a medium may take many forms, including but not limited to non- volatile media, volatile media, and transmission media. Non-volatile media may include, for example, optical or magnetic disks. Volatile media may include dynamic memory. Transmission media may include coaxial cables, copper wire and fiber optics. Transmission media may also take the form of acoustic, optical, or electromagnetic waves, such as those generated during radio frequency (RF, e.g., using an RFID tag) and infrared (IR) data communications. Common forms of computer-readable media include, for example, a diskette, hard disk, magnetic tape, or other magnetic medium, a CD-ROM, CDRW, DVD, or other optical medium, a RAM, a PROM, and EPROM, a FLASH-EPROM, or other memory chip or cartridge, a carrier wave, or other medium from which a computer can read.

"Electronically transmitting the digital wellness landscape" refers to the act of conveying the digitized anatomical information as electromagnetic radiation through an through wires, coaxial cables, dielectric slabs, optical fibers, electric power lines, and waveguides or wireless media to a receiver or storage device, which may reside at a site remote from that at which the digital wellness landscape originates. Similarly, digitized wellness information may be sent from a receiver or storage device to a site at which a digital wellness landscape can be obtained, and/or to a site at which a maintenance plan / treatment course can be prescribed by a clinician / physician / dentist.

"Data compression" refers to the process of encoding information using fewer bits (or other information-bearing units) than an unencoded representation would use through use of specific encoding schemes.

"Securing" or "security encoding" refers to the process of encrypting information for protection of the subject's personal medical and dental history and digital wellness landscape information.

"Initial digital wellness landscape" refers to the sum of all the data points in a first, untreated or restored state before the analysis and design of a subject's wellness treatment plan in accord with the present methods and systems. "Restored digital wellness landscape" refers to the treated or restored state that is a physiologically or medically desired, improved state of global wellness achieved during or subsequent to completion of the treatment plan designed in accord with the present methods and systems. "Intermediate wellness state" or "intermediate state" refers to the subject's state after treatment designed in accord with the methods and systems described herein, where the treatment adjusts one or more medical or dental health parameters to achieve a wellness status that is different from the initial digital wellness landscape status, yet is not necessarily at the desired, ideal restored state of global wellness.

An image of a subject can be obtained using a means of imaging selected from, for example, magnetic resonance imaging (MRI), computed tomography (CT), radiologic imaging such as x-rays, ultrasound imaging, infrared imaging, or any variations or combinations thereof.

"Superimposition of the digital wellness landscapes" refers to placement of an image or video representing a second digital wellness landscape on or over a first image or video representing a digital wellness landscape for comparison of two or more digital wellness landscapes. In some embodiments, the superimposition of the digital wellness landscape images aligns one or more data points in each image. As can be appreciated, superimposition of two images permits assessment of differences between an initial and an intermediate or a restored wellness state, and informs the measurements and/or calculations for design of global wellness plan / map toward maintenance or treatment.

"Simulation of a movement path" or "defining one or more movements of any data point to move from an initial state to the desired restored state" refers to the process of measuring or calculating the direction / course / path of actions (i.e., a series of behavioral steps) needed for the subject to get from an initial or intermediate wellness state at a given time point to an intermediate or restored state at a later time. The movements can be a distance in one or more of the X, Y, Z directions, or can be angular movements around the X, Y, Z axes.

"Six degrees of freedom" or "6DoF" refers to movement in three dimensional space, i.e., the ability to move forward/backward, up/down, left/right (translation in three perpendicular axes) combined with rotation about three perpendicular axes (yaw, pitch, roll). As the movement along each of the three axes is independent of each other and independent of the rotation about any of these axes, the motion has six degrees of freedom. In the context of the present disclosure, 6DoF typically refers to the movement of one or more of the subject's medical or dental data points from intial or intermediate wellness state at a given time point to an intermediate or restored state at a later time.

A "signal" refers to an electronic event, message, or data packet that can carry varying quantities of information and which is transmitted between computational processes. As used herein, a signal can be sent from a computer running an associated software program, to another computer, which can process the signal and which can then send a signal to one or more receiving parties, e.g., patient, doctor or dentist.

"Timer for scheduling a subsequent obtaining of an intermediate wellness state at a given time point to an intermediate or restored state at a later time or follow-up appointment" refers to a step of the method in which a desired length of time of treatment with the wellness program / plan has passed and the subject's wellness state is reassessed for design of the next step in treatment. For example, the timer can prompt the user (patient/clinician/physician/dentist) to schedule an appointment with the treating clinician, physician or dentist to reassess and monitor progress or to design a new treatment plan.

"Treatment plan" refers to the design of one or more courses of medications, surgical treatments, and/or changes in behavior to achieve a desired global state of wellness.

"Labeled in order of use" refers to markings on the output treatment plan that indicate the sequential order in which the prescribed treatment steps are to be performed.

"Optional" or "optionally" refers to a subsequently described circumstance that may or may not occur, so that the scope of the embodiment includes instances where the circumstance occurs and instances where it does not.

"Ameliorating" or "ameliorate" refers to any indicia of success in the treatment of a pathology or condition, including any objective or subjective parameter such as abatement, remission or diminishing of symptoms or an improvement in a subject's physical or mental well-being. Amelioration of symptoms can be based on objective or subjective parameters; including the results of a physical examination and/or a psychiatric evaluation.

Having a "predisposition to develop a disease or disorder" means that a subject having a particular genotype and/or haplotype has a higher likelihood than one not having such a genotype and/or haplotype for developing a particular disease or disorder.

"Associated" refers to coincidence with the development or manifestation of a disease, condition or phenotype. Association may be due to, but is not limited to, genes responsible for housekeeping functions whose alteration can provide the foundation for a variety of diseases and conditions, those that are part of a pathway that is involved in a specific disease, condition or phenotype and those that indirectly contribute to the manifestation of a disease, condition or phenotype.

When a range of values is provided, it is understood that each intervening value, to the tenth of the unit of the lower limit unless the context clearly dictates otherwise, between the upper and lower limits of that range is also specifically disclosed. Each smaller range between any stated or intervening value in a stated range and any other stated or intervening value in that stated range is encompassed by the disclosure. The upper and lower limits of the smaller ranges can be independently included or excluded in the range, and each range where either, neither or both limits are included in the smaller ranges is also encompassed by the disclosure, subject to any specifically excluded limit in the stated range. Where the stated range includes one or both of the limits, ranges excluding either or both of those included limits are also included.

The Bale-Doneen method and compositions for predicting cardiovascular events are disclosed in US Patent Application publication 2012129708A1. Also described are methods, systems, devices, panels, and software for determining values for two or more markers in order to characterize a subject's risk of developing cardiovascular disease or experiencing a complication thereof (e.g., within the ensuing one to three years), and for identifying subjects in need of preventative therapy (e.g., statins). In certain embodiments, the markers are selected from: hs-CRP, ACR, Lp-Pla2, MPO, fibrinogen, KIF6, and F2 isoprostanes.

Russian patent 2336803 entitled "Method for hypertension and diabetes mellitus detection at dental reception" describes examination of a patient's mouth cavity to detect any carious cavities, fillings, extracted teeth and to estimate parodentium tissue condition. Then condition of organs and tissues of the mouth cavity are characterized according to four clinical indices ("PMA", " 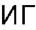 P-Y", " 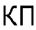 Y", " 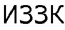 "), each of which is ranked from 1 to 3 depending on the index value. Then numbered estimation is carried out as total score of the ranks in four indices. The total score value less than 8 means low risk of patient's having diabetes mellitus (DM) or hypertension (H). The total score value equal to 9-10 indicates substantial risk of having DM or H. The total score value equal to 11-12 shows higher probability of having H or DM. To determine the diagnostic indices, five groups of people of different age are examined, the 1st group includes patients having H, the 2nd group consists of patients having DM, the 3d group - patients with pyelonephritis, the 4th group - patients having dental deposit in the mouth cavity, the 5th group - "control group" - people with the same physical status "almost healthy" without any diseases of parodentium and tunica mucosa of mouth.

Russian patent 2321335 describes a method for pre-medicating cardiovascular abnormality patients within the framework of out-patient visit to dentist, involving estimating patient health state condition based on diagnostic cardiac rhythm variability data as intersystolic interval duration mean square deviation. The diagnostic index being equal to or less than 20 ms, Tophysopam is to be orally introduced at a dose of 50 mg 30 min before surgical intervention. The diagnostic index value being greater than 20 ms and less than 50 ms, Tophysopam is to be introduced at a dose of 25 mg 30 min before surgical intervention. The method is said to enhance effectiveness of anesthesia and reduce risk of adverse vegetative cardiovascular system responses.

PCT Publication WO2000064334 describes a non-invasive glucose monitoring device includes a mechanism for stimulating salivary glands secretion of saliva into oral fluid prior to collecting a sample of the oral fluid. A mechanism is provided for detecting the amount of glucose in the sample, a mechanism also being provided for quantitating blood glucose level based on the amount of glucose detected. A method of non-invasively monitoring glucose includes the steps of stimulating salivary glands secretion of saliva into oral fluid, collecting a sample of the oral fluid, detecting an amount of glucose in the sample, and then quantitating the blood glucose level based on the amount of glucose detected. These methods and devices are used to diagnose individuals with glucose levels that indicate a diabetic disease state.

US Patent Application publication 20120321724 describes a method of measuring in vivo nitric oxide (NO) and nitrite levels in individuals by providing a salivary nitrite test substrate, testing salivary nitrite levels with the test substrate, measuring nitrite levels detected in the testing; and correlating the measured nitrite levels with in vivo nitric oxide bio-availability.

US Patent No. 5,500,374 discloses a rapid, simple and reliable method and device for diagnosing diabetes mellitus under no influence of diet. The method for diagnosing diabetes mellitus involves injecting a saliva sample from a patient suspected of having diabetes mellitus into a stationary phase in a separation column, thereafter eluting a phosphate buffer as an eluent to separate analytes contained in the saliva sample, subsequently detecting the individual separated analytes with a UV detector and electrostatic ion chromatography to produce a chromatogram, and then diagnosing diabetes mellitus based on the presence or absence of a chromato-peak of the diabetes mellitus-specific component. A zwitterionic stationary phase is employed as the stationary phase and contains a support carrier and a zwitterionic charged layer formed by coating a compound having an ammonium salt portion, a sulfonate ion portion or carboxylate ion portion on the surface of the support carrier.

US Patent Application publication 20100167306 describes a rapid immunochromatographic assay for measuring the ratio of glycated albumin to total albumin in saliva. Patients with diabetes have elevated levels of glucose in their blood that can react with plasma albumin to form glycated albumin. The amount of glycated albumin formed is directly correlated with the level of plasma glucose that the albumin has been exposed to over a period of time. Saliva albumin is derived from plasma albumin and therefore contains glycated and non-glycated albumin fractions that can be measured. The ratio of glycated albumin to total albumin in saliva will provide an indication of the average amount of protein glycation that occurred over the preceding 2-3 week period. The test is performed using a disposable strip that contains the testing reagents and the results are measured in a measuring instrument that automatically reads, calculates and displays the final result. The results of tests performed over a period of time are stored in the instrument's memory and presented in a numerical or graphical format so that the individual's glycated albumin level can be monitored over time.

Also possibly useful in combination with the present disclosure are US Patent Application Publication No. 2002107448 disclosing an oral probe device employing diffusereflectance spectroscopy; US Patent Application Publication No. 2009264507 disclosing oral wound healing; and PCT Publication WO9906827A2 disclosing a saliva collection device.

US Patent No. 8,163,502 to Denny et al*.* describes a saliva-based test employing a panel of lectins (DSL (*datura stramonium*), ECL (*erythrina cristagalli*), PSA (*pisum sativum*)*,* WGA (*triticum vulgaris*), UEA I (*ulex europaeus*)*,* MAL I (*maackia amurensis*), MAA (*maackia amurensis*)*,* PNA (*arachis hypogaea*), AAL (*aleuria aurantia*)*,* LTL (*lotus tetragonolobus*), MAL II (*maackia amurensis*), JAC (Artocarpusintegrifolia), LEL (*lycopersicon esculentum*)*,* SNA (*sambucus nigra*), PTL I (*psophocarpus tetrogonolobus*), ACL (*amaranthus caudatus*), GSL II (*griffonia simplicifolia*), VVA (*vicia* villosa), BPL (*bauhinia purpurea*), WFL (*wisteria floribunda*), SJA (*sophora japonica*)*,* MPL (*maclura pomifera*), GNL (*galanthus nivalis*), HHL (*hippeastrum hybrid*), CCA (*canavalia ensiformis*), NPL (*narcissus pseudonarcissus*), STL (*solanum tuberosum*), PHA-L (*phaseolus vulgaris*), PHA-E (*phaseolus* vulgaris), GSL I (*griffonia simplicifolia*)*,* DBA (*dolichos biflorus*), HMA (*homarus americanus*), EEA (*euonymous europaeus*), LPA (*limulus polyphemus*), and PTL II (*psophocarpus tetrogonolobus*) for predicting the risk of oral diseases and such as dental caries, periodontal disease, mucosal infections, oral and pharyngeal cancers and precancerous lesions, HIV and osteoporosis and oral bone loss. Similarly, PCT Publication WO2004089187 describes methods, test devices, and diagnostic kits for predicting, assessing, and diagnosing the risk of a disease using salivary analysis, comprising providing a whole (unfractionated) saliva sample from a subject, contacting an aliquot of the saliva with one or more lectins under conditions allowing lectins to bind to a lectin-binding component of said saliva, detecting the amount of bound lectin, and comparing the amount of bound lectin to the amount known to bind a saliva sample from a control patient, to predict the risk of a disease in the subject; and US Patent Application Publication No. US 20030040009 to Denny et al describes compositions, methods and diagnostic kits for detecting a disease based on measurement of salivary mucins.

US Patent Application Publication 20110288013 describes methods of reducing risk of developing cardiovascular complications in diabetic patients. Specifically, the invention relates to the use of haptoglobin genotyping for determining the importance of maintaining tight glycemic control in diabetic subjects expressing Hp 2-2 allele.

US Patent No. 6,309,827, assigned to OraSure Technologies Inc., describes a rapid and convenient method of simultaneous collection of oral fluid including saliva and/or mucosal transudate for testing of both genomic and non-nucleic analytes. Collection of oral fluids entails minimal invasion of privacy, is convenient, relatively safe, and can be accomplished rapidly with ease. In addition, in patients where only small volumes of blood can be drawn, for example, newborns and the elderly, adequate volumes for testing can be obtained from saliva or mucosal transudate, and diagnostic information can be gleaned from a single sample on a bibulous pad by differential extraction of the diagnostic information from the genomic information. A PCR assay on the contents of the bibulous pad provides results comparable in reliability, specificity, and sensitivity to the best available serum (blood) based assays.

Access Genetics, Alticor Inc., and Interleukin Genetics Inc. are assignees on several illustrative publications. For example, PCT Publication WO03027236 and European Patent Publications EP 1578904 and EP 2071040 describe methods, systems and kits for genetic testing, especially for situations in which the procurement of genetic source material occurs at a different location than the testing of the material and the interpretation of the results. The disclosed kits and methods relate to the diagnosis of cardiovascular disorders, including an occlusive disorder, fragile plaque disorder, and restenosis disorder. Similarly, US Patent No. 8,594,948 discloses an apparatus and methods for practicing telemedicine in the form of software systems acting over a network and kits containing laboratory supplies and equipment to organize the laboratory operations and interpret the results of molecular diagnostic testing. At least two computers in communication over the internet or other network are used, a remote computer located at a remote site and a central server located at a central site. The remote site may be geographically distant from the central site. A specimen is procured from a patient proximate to the remote site. Laboratory operations are conducted on the specimen at the remote site. The laboratory data resulting from the laboratory operations is interpreted by an expert reviewer who may be located at the central site, and a report is then transmitted back to the remote site. US Patent Application Publication No. 2005095628 discloses a program for regulating a health condition including one or more assessments including a genetic test, biomarker test, lifestyle assessment, a personalized intervention and a follow up test for monitoring a subject's health condition. US Patent Application Publication Nos. 2009216559 and 2010136561 describe systems for providing anonymous access to health information, and genetic markers used to establish personalized weight-loss programs based on a metabolic genotype, respectively.

US Patent Nos. 7,827,039 and 7,998,070 having Patrick Gentempo as a named inventor are directed to wellness program. US 7,998,070 describes a method of quantifying neurospinal function and determining a neurospinal functional index (NSFI), to provide a single index quantifying a plurality of neurospinal functions, for example, algometry, range of motion, electromyography, thermography, and heart rate variability. Embodiments of the NSFI are said to be useful in providing an objective index of a patient's neurospinal function, for example, in chiropractic assessments.

US 7,827,039 describes a system that is said to generate one or more of a plurality of wellness programs for a user. Personal data items are entered into a computing system corresponding to a personal profile of the user. A selected one or more of the plurality of wellness programs desired by the user is identified. Selected ones of the plurality of personal data items are then extracted in response to the selected one of the plurality of wellness programs. Then a corresponding plurality of wellness program data elements in response to the selected ones of the plurality of personal data items is determined. The selected plurality of wellness program data elements are then assembled to form a customized wellness program responsive to the step of identifying a selected one of the plurality of wellness programs and the selected ones of the plurality of personal data items. The customized wellness program is recorded on media for the user. The invention relates generally to systems and programs for improving the well-being of an individual, and more particularly, to a computer-implemented system for generating a highly customized program that improves the psychological, spiritual, and/or physical well-being of an individual.

US 7,827,039 states that, absent direct intervention by a professional, such as a psychologist, clergyman, trainer, nutritionist, or doctor, systems that address the psychological, spiritual, and/or physical well-being of an individual are generally not responsive to needs that are specific to the individual. Instead, such systems apply generalized concepts that may be specific to the system to all who seek to obtain the benefits that the particular system purports to provide. Thus, for example, conventional self-help, or motivational, programs set forth overall goals to be achieved and a general methodology for achieving same, but generally leave it up to the individual to apply the principles of the program to his or her specific requirements. A self-help program that can be obtained commercially on cassette, DVD, or CD media will not have provision for receiving data specific to a user, or a facility for customizing the program to the needs of the particular user.

US 7,827,039 further states that it is an object of the patent to provide a system for generating one of a plurality of wellness programs for a user. The system includes the steps of: entering a plurality of personal data items into a computing system corresponding to a personal profile of the user; identifying a selected one of the plurality of wellness programs desired by the user; extracting selected ones of the plurality of personal data items in response to the selected one of the plurality of wellness programs; selecting a plurality of wellness program data elements in response to the selected ones of the plurality of personal data items; assembling the selected plurality of wellness program data elements to form a customized wellness program responsive to the step of identifying a selected one of the plurality of wellness programs and the selected ones of the plurality of personal data items; and recording the customized wellness program on media. Prior to performing the step of entering a plurality of personal data items into a computing system there is provided the step of filling out a personal profile of the user. Such a profile may include medical and lifestyle data. In addition, the profile may include birth date information and a child number (i.e., second of four children). The resulting customized wellness program can be presented to the user as a printed item, but preferably should be in the form of a CD or DVD that include picture, video, audio, and text data. The plurality of wellness program data elements for inclusion in the customized wellness program may include an audio file, which may be a spoken presentation, background music, and audio effects. US 7,827,039 provides the further step of selecting one of a plurality of narrator voices to be used in the customized wellness program. Additionally, such data elements may include a video file that includes a spoken presentation, visual background effects, and an illustrative presentation of an aspect of the customized wellness program, such as background information about the program, its duration, etc. In a further embodiment, the step of selecting a plurality of wellness program data elements includes the step of selecting workout video data elements responsive to the medical data and to the lifestyle data. Also disclosed is the step of selecting a plurality of wellness program data elements includes the step of selecting Chinese calendar data elements responsive to the birth date data about the user. In addition, there may be included the step of selecting Enneagram Personality data elements and/or Arevadic Body type data elements responsive to the birth date data about the user.

US 7,827,039 describes a customized relaxation program, including the step of user reviewing a menu of positive affirmations includes the step of reviewing a menu of topics of positive affirmations, the topics corresponding to self-esteem, self-confidence, stress management, self-control, etc. Also provided is a system for generating a program for a client, the system having the steps of: filling out a questionnaire containing inquiries regarding a plurality of physical and lifestyle aspects of the client; obtaining a plurality of physical measurements of the client; obtaining a plurality of biochemical characteristics of the client; entering into a computing system a first data element that is responsive to responses in the questionnaire; entering into the computing system a second data element that is responsive to the client's physical measurements; entering into the computing system a third data element that is responsive to the client's biochemical measurements; entering into the computing system a fourth data element that is responsive to the client's psychological measurements; calculating a wellness quotient of the client that is responsive to the first, second, third, and fourth data elements, the wellness quotient being representative of an overall wellness condition of the client; selecting an item of output information from a plurality of items of information in response to the wellness quotient; and recording output data on media that is responsive to the selected output information.

US 7,827,039 further describes a the step of filling out a questionnaire includes the steps of responding to inquiries relating to the client's physical characteristics, to produce a physical questionnaire data element; responding to inquiries relating to the client's biochemical characteristics, to produce a biochemical questionnaire data element; and responding to inquiries relating to the client's psychological characteristics, to produce a psychological questionnaire data element. Physical questionnaire scoring produces a physical questionnaire score value that is responsive to the physical questionnaire data element. Biochemical questionnaire scoring produces a biochemical questionnaire score value that is responsive to the biochemical questionnaire data element. Similarly, psychological questionnaire scoring produces a psychological questionnaire score value that is responsive to the psychological questionnaire data element. In a similar manner, physical measurement scoring produces a physical measurement score value that is responsive to the second data element, biochemical measurement scoring produces a biochemical measurement score value that is responsive to the third data element, and psychological measurement scoring produces a psychological measurement score that is value responsive to the fourth data element. The physical questionnaire score value, the biochemical questionnaire score value, the psychological questionnaire score value, the physical measurement score value, the biochemical measurement score value, and the psychological measurement score value can be subjected to adjustment in response to a predetermined multiplier value.

US 7,827,039 describes a method comprising the steps of: entering into a computer system, by a user, responses to a questionnaire containing inquiries regarding a plurality of physical and lifestyle aspects of the human client; wherein the responses to the inquiries relate to the human client's physical characteristics, biochemical characteristics, and psychological characteristics; entering into the computer, by the user, a plurality of the human client's physical measurements, biochemical measurements, and psychological measurements; calculating, by the computer system, a transformed physical score ("Pht") based on the entered questionnaire responses relating to the human client's physical characteristics and the human client's entered physical measurements, a transformed biochemical score ("Bt") based on the entered questionnaire responses relating to the human client's biochemical characteristics and the human client's entered biochemical measurements, and a transformed psychological score ("Pst") based on the entered questionnaire responses relating to the human client's psychological characteristics and the human client's entered psychological measurements; calculating, by the computer system, a wellness quotient ("Wq") of the human client, the wellness quotient being representative of an overall wellness condition of the human client, wherein the wellness quotient ("Wq") is calculated in accordance with the relationship: Wq=(Pht)^{0.4}(Bt)^{0.4}(Pst)^{0.2}; selecting and outputting, by the computer system, an item of output information from a plurality of items of information based on the calculated to the wellness quotient; and recording, by the computer system, the item of output information on media responsive to the selecting of output information.

US 7,827,039 describes the further steps of: waiting a predetermined period of time; entering into a computer system, by a user, new responses to a questionnaire containing inquiries regarding a plurality of physical and lifestyle aspects of the human client; wherein the new responses to the questionnaire relate to the human client's physical characteristics, biochemical characteristics, and psychological characteristics; entering into the computer system, by the user, a plurality of the human client's new physical measurements, biochemical measurements, and psychological measurements; calculating, by the computer system, a new transformed physical score ("Pht") based on the newly entered questionnaire responses relating to the human client's physical characteristics and the human client's entered physical measurements, a new transformed biochemical score ("Bt") based on the newly entered questionnaire responses relating to the human client's biochemical characteristics and the human client's entered biochemical measurements, and a new transformed psychological score ("Pst") based on the newly entered questionnaire responses relating to the human client's psychological characteristics and the human client's entered psychological measurements; calculating, by the computer system, a new wellness quotient ("Wq") of the human client, the wellness quotient being representative of an overall wellness condition of the human client, wherein the new wellness quotient ("Wq") is calculated in accordance with the relationship: Wq=(pht)^{0.4}(Bt)^{0.2}; selecting and outputting, by the computer system, an item of output information from a plurality of items of information based on the calculated to the wellness quotient; and recording, by the computer system, the item of output information on media responsive to the selecting of output information. The computer system may further be configured to: receive new entered responses to a questionnaire containing inquiries regarding a plurality of physical and lifestyle aspects of the human client, wherein responses to the inquiries relate to the human client's physical characteristics, biochemical characteristics, and psychological characteristics; receive the human client's new entered physical measurements, biochemical measurements, and psychological measurements; calculate a new transformed physical score ("Pht") based on the newly entered questionnaire responses relating to the human client's physical characteristics and the human client's entered physical measurements, a new newly transformed biochemical score ("Bt") based on the entered questionnaire responses relating to the human client's biochemical characteristics and the human client's entered biochemical measurements, and a new transformed psychological score ("Pst") based on the newly entered questionnaire responses relating to the human client's psychological characteristics and the human client's entered psychological measurements; calculate a new wellness quotient ("Wq") of the human client, the wellness quotient being representative of an overall wellness condition of the human client, wherein the wellness quotient ("Wq") is calculated in accordance with the relationship:

Wq=(Pht)^{0.4}(Bt)^{0.4}(Pst)^{0.2}; and forming a customized revised wellness program based on the inputted new calculated wellness quotient ("Wq") of the human client. The computer system can be configured to provide an item of physical exercise equipment based on the wellness quotient ("Wq"). Also provided is a computer program media product of the type having a plurality of data storage locations tangibly embodied on a computer readable medium, that when executed by a computing device performs the method steps comprising: accessing personal data items stored in a personal data storage region, the personal data items being based on personal information of a human client that is based on responses to a questionnaire containing inquiries regarding a plurality of physical and lifestyle aspects of the human client, relating to the human client's physical characteristics, biochemical characteristics, and psychological characteristics, the personal data items being tangibly embodied in the personal data storage region, and having been precalculated by a computing system as a transformed physical score ("Pht") based on the entered questionnaire responses relating to the human client's physical characteristics and the human client's entered physical measurements, a transformed biochemical score ("Bt") based on the entered questionnaire responses relating to the human client's biochemical characteristics and the human client's entered biochemical measurements, and a transformed psychological score ("Pst") based on the entered questionnaire responses relating to the human client's psychological characteristics and the human client's entered psychological measurements; and accessing and presenting to the human client a pre-computed wellness program customized for the human client in response to the personal information of the human client, the wellness program being based on a pre-computed wellness quotient ("Wq") and tangibly embodied in a wellness data storage region, the wellness quotient having been pre-computed in accordance with the relationship: Wq=(Pht)^{0.4}(Bt)^{0.4}(Pst)^{0.2}. The wellness program can include a customized relaxation program and a personalized guided visualization program. The wellness program can be a multimedia presentation. The wellness program can include exercise data responsive to a workout program based on the personal information of the human client and the pre-computed wellness quotient. The wellness program can include nutritional data responsive to a nutrition program determined in response to the personal information of the human client and the pre-computed wellness quotient.

### CVD:

US Patent No. 7,445,886 discloses that macrophage migration inhibitory factor (MIF) is a clinically useful biochemical marker of cardiovascular risk. Risk assessment includes the step of detecting in the blood of a person MIF concentration as a marker of cardiovascular risk for the person. The method may further comprise the step of assigning to the person a cardiovascular risk metric proportional to the MIF concentration, and/or prescribing for the person a cardiovascular treatment modality in accordance with the MIF concentration. The method is useful as a primary screen, and may be used in conjunction with or as a substitute for additional tests, such as a stress test, CRP assay, LDL assay, etc. The detecting step may be repeated over time intervals and/or treatment to monitor change in cardiovascular risk for the person over time and/or treatment.

PCT Publications WO2013020870 and WO2013036285 describes two clinical validation Studies to demonstrate that determining an aggregate biomarker risk score, including levels of C-reactive protein (CRP), a fibrin and fibrinogen degradation product (FDP) marker (including a mixture of multiple FDPs), and Heat Shock Protein 70 (HSP70), can be used to assess or indicate future risk of adverse events in subjects. Cytomegalovirus antibody (anti-CMV Ab) and antibody to Heat Shock Protein 60 (anti-HSP60) also were assessed. CRP was measured using a high-sensitivity CRP (hs-CRP) assay (GenWay Biotech., Inc., catalog number 40-052- 115042). The FDP marker was measured using the DR-70^{®} ELISA assay. In the disclosed method, 1500 subjects were selected in a case-control study from the Emory Cardiology Biobank, a registry of patients undergoing cardiac catheterization; included those: (1) with angiographic CAD who suffered death, Ml at 2 years of follow-up after enrollment (n=200 subjects); (2) with angiographic CAD without MI or death (n=800) of which 300 subjects underwent revascularization during 2 years of follow up after enrollment; and (3) with non- significant CAD based on angiography (n=500). Groups were matched for age, gender and cardiovascular risk factors using propensity scoring (Rosenbaum and Rubin, Biometrika, 1983, 70(1), 41-55; *See* en.wikipedia.org/wiki/Propensity_score matching). Serum biomarkers were measured using ELISA (GenWay^{®} Inc.). Cox proportional hazard survival analyses were performed with models further adjusted for age, BMI, sex, race, smoking, diabetes, and hypertension. Data were analyzed as continuous variables and with cut points assigned based on accepted values. Levels of CMV antibody and anti-HSP60 were not significantly associated with any outcomes. For primary outcomes of death and MI at two years, with adjustments for standard cardiovascular risk factors, the association for each biomarker modeled continuously demonstrated a hazard ratio (HR) of 1.55 (p<0.0001). Modeling with cut points revealed hazard ratios of 1.89 (p <0.001) for CRP> 3.0 mg/L; HR of 1.42 (p=0.005) for HSP70 when detectable; and HR of 2.04 (p<0.001) for FDP >1.0 µg/ml. HRs for the primary outcome using categorical risk scores calculated using all 3 significant biomarkers in aggregate revealed 1.68 (p= 0.008) for 1 biomarker, 2.64 (p <0.0001) for 2 biomarkers, and 3.76 (p <0.0001) for 3 biomarkers elevated. These results provided the basis for a scoring system and for an aggregate score. The results showed that the measurement, particularly in the aggregate, of levels of CRP, an FDP marker, and HSP70, is a strong predictor of future risk of death and MI in patients without known CAD. An additional study was carried out, with four patient groups: Cohort A - angiographically confirmed significant CAD (> 50 % coronary stenosis) - with Ml or death; Cohort B - Angiographically confirmed significant CAD without MI or death; Cohort C - Angiographically confirmed insignificant CAD (< 50 % coronary stenosis as determined angiographically) with Ml or death; and Cohort D - Angiographically confirmed insignificant CAD (< 50 % coronary stenosis as determined angiographically) without MI or death -, 3,800 subjects total. 1,500 subjects were used in an initial testing study; 2,300 subjects were used in a subsequent validation study. Blood was collected for sampling, all drawn prior to catheterization. Presence of severity and coronary artery disease was documented with angiograms. Data collection period was present, with mean follow-up of 2.75 years. The serum levels of CRP, HSP70 antigen, FDP marker (as described above), and CMV antibody were measured. CRP was measured using a high-sensitivity CRP (hs-CRP) assay, as described in (Gen Way Biotech., Inc., catalog number 40-052-115042). For CAD participants only, after excluding acute MI and transplants, and missing on previous MI, PCI history or biomarker risk score, there were 1637 participants in the analysis; 190 of these participants had had death or MI events. * For total participants, after excluding acute MI and transplants, and missing on previous MI, PCI history or biomarker risk score, there were 2951 participants in the analysis; 296 of these participants had had death or MI events. Diabetes was defined as medication use or glucose >=200 mg/dL. Hypertension was defined as medication use, SBP>140 or DBP>90 0 CRP > 3.0 mg/L, HSP70 > 0 ng/mL, FDP > 1.0 ug/mL Cox proportional hazard survival analyses were performed with models further adjusted for age, sex, race, smoking, diabetes, and hypertension. CRP was considered "elevated" if greater than 3.0 mg/mL. HSP70 was considered "elevated" when detectable. The FDP marker was considered "elevated" when greater than 1.0 microgram/mL. In patients with significant CAD, the hazard ratios for future risk of death or MI using categorical risk scores calculated using various numbers of biomarkers in aggregate were 2.08 for 1 biomarker elevated, 3.38 for 2 biomarkers elevated, and 5.37 for 3 biomarkers elevated. Numbers in parentheses indicate HR (hazard ratio) confidence intervals. In patients with insignificant CAD, the hazard ratios for future risk of death or MI using categorical risk scores calculated using various numbers of biomarkers in aggregate were 1.40 for 1 biomarker elevated, 4.11 for 2 biomarkers elevated, and 5.50 for 3 biomarkers elevated. Numbers in parentheses indicate HR (hazard ratio) for confidence intervals.

US Patent No. 7258994 to Vojdani and US Patent Application Publication No. 2006094073 disclose saliva immunoassays for detection of antibodies indicative of cardiovascular disease.

US Patent No. 7,651,868; US Patent Application Publication No. 20080300798 and PCT Publications WO2005059551, WO2008131039 and WO2011022628 describe a system for the rapid detection and characterization of analytes in saliva, including a light source, a sensor array, and a detector. The sensor array is formed from a supporting member, in which a plurality of cavities may be formed. A series of chemically sensitive particles, in one embodiment, are positioned within the cavities. The particles may produce a signal when a receptor, coupled to the particle, interacts with the cardiovascular risk factor analyte and the particle-analyte complex is visualized using a visualization reagent. Using pattern recognition techniques, the analytes within a multi-analyte fluid may be characterized. In an embodiment, each cavity of the plurality of cavities is designed to capture and contain a specific size particle. Flexible projections may be positioned over each of the cavities to provide retention of the particles in the cavities.

European Patent Application EP 2186913 A3 describes genetic polymorphisms that are associated with cardiovascular disorders, particularly acute coronary events such as myocardial infarction and stroke, and genetic polymorphisms that are associated with responsiveness of an individual to treatment of cardiovascular disorders with statin. In particular, the invention relates to nucleic acid molecules containing the polymorphisms, variant proteins encoded by such nucleic acid molecules, reagents for detecting the polymorphic nucleic acid molecules and proteins, and methods of using the nucleic acid and proteins as well as methods of using reagents for their detection.

US Patent No. 8,030,097 to Xu, et al*.,* discloses a method and apparatus for immunoassay and measurement of lipocalin-2 in body fluids for prediction of heart and stroke risk, and determination of the likelihood that certain individuals will benefit to greater or lesser extent from the use of certain treatments designed to prevent CVD.

PCT Publication WO2012050513 describes a method for identifying risk of cardiovascular disease or atherosclerosis by analyzing an individual's oral microbiota such as bacterial genera *Veillonella, Streptococcus, Chlamydia, Pseudomonas luteola, Staphylococcus, Proprionibacterineae* and *Burkholderia.* Antibacterial agents that inhibit growth of these oral flora include, but are not limited to, metronidazoles, flurorquinolones, penicillins, cephalosporins and tetracyclins. Phyla found in body habitat include Firmicutes, Bacteroidetes, Actinobacteria, Fusobacteria, Proteobacteria, TM7, Spirochaetes, SRI, Tenericutes, Deinococcus-Thermus, Acidobacteria, Gemmatimonadetes and Chloroflexi.

Korean Patent Application KR 20100061438 describes microarrays for detection and identification of pathogenic microorganisms associated with periodontal diseases and for use in methods for diagnosis of infectious oral diseases. Disclosed is a target DNA of an ITS (internal transcribed spacer) region which contains one of several specific nucleotides (or a part thereof) for detecting gram-positive bacteria and primer sets and PCR kits for amplifying bacteria relating to periodontal diseases and containing the oligonucleotide. The method for detecting the bacteria relating to periodontal diseases comprises isolating nucleic acids from a sample; amplifying a target DNA in the nucleic acid; hybridizing the amplified DNA with the probe of the microarray; and detecting a hybridization signal.

### Diabetes:

PCT Publication WO 98/54359 describes the IL-1 gene cluster found to have polymorphisms associated with inflammatory diseases, including diabetes and periodontal disease.

PCT Publication WO WO2012085674 describes a method to predict, in subjects affected by type-2-diabetes (T2D), the probability of developing complications related to the disease. The invention involves (1) identification of genetic features such as single nucleotide polymorphisms (SNPs) for the establishment of a patient profile that can be used for prediction of complications associated with T2D. Signature profiles comprising a combination of SNPs which have greater predictive value for prognosticating particular types of complications, such as, stroke, myocardial infai'ction and kidney complications associated with T2D are further described. Compositions and kits that can be used with a set of complementary phenoiypic markers to evaluate the risk for an individual affected by T2D to develop complications related to the disease and to evaluate the likelihood that an individual affected by type 2 diabetes type will benefit from treatments that collectively aim to reduce the risk of developing such complications.

WO2012085674 further describes existing treatments for diabetes: therapeutic agents or compounds currently used for the treatment of T2D and T2D-related complications are combined with one or more known therapeutic agents used to treat T2D comprising I. oral antidiabetics including biguanid derivatives such as 1) metformin, 2) buformin, insulin secretagogues sach as 1) sulphonylurea derivatives such as tolbutamide, glibenclamide, gliclazide, glipizide,, glimepiride, gliquidone; 2) meglitinides such as repaglinide, nateglinide; 3) alpha- glucosidase inhibitors such as acarbose, migliiol; 4) thiazolidinediones such as rosiglitazone and pioglitazone; 5) other defined by World Health Organisation - The Anatomical Therapeutic Chemical (ATC) classification system; II. insulin such as i)insuUn glargine, ii) insulin aspart, iii) insulin lispro, iv) insulin glulisine; v) insulin deiemir; and agents known do decrease and or prevent diabetes related complication, such as high blood pressure, i) converting enzyme inhibitors, ii) angiotensin receptor blockers, iii) direct renin inhibitors, iv) endothelin antagonists, v)diuretics, vi)beta blockers, vii)alpha blockers, viii) inhibitors of phospodiesterase 5 a and the combinations thereof. A number of genes and genetic polymorphisms were tested for their association to diabetic nephropathy, either because of their reported relevance in metabolic and signaling pathways connected to pathophysiology of diabetic complications (functional candidates) or combination of the former with their genomic position under peak of ascertained linkage (positional candidates), or as a result of genome- wide association studies. Genes for which an association was found with diabetic nephropathy include 5, 10-methylenetetrahydro folate reductase (MTHFR), natriuretic peptide precursor A (NPPA). solute carrier family 2 member 1 (facilitated glucose transporter SLC2A1), lamin A/C (LMNA), retinoid X receptor gamma (RXRG), interleukin 1 receptor antagonist (IL1RN), ghrelin/obestatin preprohormone (GHRL), peroxisome proliferator-activated receptor gamma (PPARG), chemokine receptor 5 (CCR5), angiotensin II receptor type 1 (AGTR1), solute carrier family 2 member 2 (facilitated glucose transporter SLC2A2), adiponectin (ADIPOQ), fatty acid binding protein 2 (FABP2), glutamine-fructose-6-phosphate transaminase 2 (GFPT2), advanced glycosylation end product-specific receptor (ACER), lymphotoxin alpha (LTA), vascular endothelial growth factor A (VEGF), ectonucleotide pyrophosphatase/phosphodiesterase 1 (E PP1), SMT3 suppressor of mif two 3 homolog 4 (small ubiquitin-like modifier 4 protein SUM04), estrogen receptor 1 (ESR1), superoxide dismutase 2 (SGD2), neuropeptide Y (NPY), engulfment and cell motility 1 (ELMOI), insulinhke growth factor binding protein 1 (IGFBP1 ), epidermal growth factor receptor (EGFR). paraoxonase 1 (PONI). aldo-keto reductase family 1 , member B 1 (AKR 1 B 1 ), caldesmon 1 (CALD1), nitric oxide synthase 3 (NGS3), hpoprotein lipase (LPL), Pvtl oncogene homolog MYC activator (PVT1), insulin (INS), xylosyltransferase I (XYLT), protein kinase C beta 1 (PRKCB 1), solute carrier family 12 (SLC12A3), haptoglobin (HP), chemokine ligand 2 (CCL2), angiotensin I converting enzyme (ACE), meprin A, beta (MEPIB), camosine dipeptidase 1 (CNDP 1 ),intercelluiar adhesion molecule 1 (ICAMI ), transforming growth factor beta 1 (TGFB I), apolipoprotein E (APOE) and superoxide dismutase 1 (SOD1), CD48 molecule (CD48), solute carrier family 35, member F3 (SLC35F3), endothelial PAS domain protein 1 (EPAS 1), low density hpoprotein -related protein IB (deleted in tumours) (LRPIB), dynein, axonemal, heavy chain 7 (DNAH7), ADAM metallopeptidase domain 23 (ADAM23), leprecan-likei(LEPRELI), human immunodeficiency virus type I enhancer binding protein 2 (HTVEP2), thrombospondin, type I, domain containing 7A (THSD7A). S - adenos ylhomocy steine hydrolase-like 2 (AHCYL2), discs, large homoiog 2 (Drosophila) (DLG2), FYVE, RhoGEF and PH domain containing 4 (FGD4), rabphihn 3A homoiog (mouse) (RPH3A). citrate lyase beta like (CLYBL), protein kinase C, eta (PRKCH), epidermal growth factor receptor pathway substrate 15 -like 1 (EPS15L1), cystatin 9 (testatin) (CST9), pericentrin (PCNT2), matrix metallopeptidase 9 (gelatinase B, 92kDa gelatinase, 92kDa type IV collagenase) ( MP9), apolipoprotein C-I (APGC1), cysteinyl-t A synthetase (CARS), chimerin (chimaerin) 2 (CHN2), ncurocalcin delta (NCALD), ectonucleotide pyrophosphatase/phosphodiesterase 1 (ENPP1), major histocompatibihty complex, class [Pi], DR beta 1 (HLA-DRB I), interleukin 8 (IL8), pleckstnn homology domain containing, family H (with MyTH4 domain) member 2 (PLEKHH2). angiotensinogen (serpin peptidase inhibitor, clade A, member 8) (AGT) and others, reviewed in Maeda, S: Genetics of diabetic nephropathy. Ther Adv Cardiovasc Dis 2, 363-71 (2008). Genes and markers in type-2 diabetes and obesity are described in US patent application publication No. 2007-0292412 to Salonen et al. Few proteomic studies have been so far conducted with the aim of identifying biomarkers of predictive value for diabetic nephropathy. Otu et al. (Diabetes Care (2007) 30:638-543) (WO 2007/056587) performed a nested case-control study on Pima Indians that allowed for identification of a 12-peak proteomic signature that was validated on a small number of individuals. However, the study did not permit the identification of the proteins from which these peaks belong and replication in other populations is needed prior to concluding the broad applicability of these biomarkers.

To date, there have been dozens of genetic variants and genome regions associated or linked to myocardial infarction, both in T2D patients as well as in non-diabetics. These have been comprehensively reviewed recently (Yamada, Y, S ichihara.T Nishida: Molecular genetics of myocardial infarction. Genomic Med 2, 7-22 (2008)) and specifically for genome- wide association studies in A Catalog of Published Genome- Wide Association Studies by The National Human Genome Research Institute (available on the world wide web at genome.gov/26525384) Johnson, AD, CJ O' Donnell: An open access database of genome-wide association results. BMC Med Genet 10, 6 (2009).

US Patent Nos. 8,216,847 and 8,216,848 describe a method for diagnosing a predisposition for diabetes, comprising determining a metabolite in a test sample of a subject suspected to have a predisposition for diabetes and comparing the metabolite to a reference to diagnose the predisposition for diabetes. Moreover, the present invention encompasses a collection of metabolites, a data collection comprising characteristic values of metabolites and a storage medium comprising the data collection. The present invention also relates to a system comprising methods or devices for comparing characteristic values of metabolites of a sample operatively linked to a data storage medium. Additionally, diagnostic methods or devices comprising a metabolite and its use for manufacture of the diagnostic method or device for diagnosing a predisposition for diabetes are provided, along with a method for identifying diabetes-related metabolites.

US Patent Nos. 7,723,050, 8,119,358 and 8,409,816 describe biomarkers associated with Diabetes, methods of using the biomarkers to determine the risk that an individual will develop Diabetes, and methods of screening a population to identify persons at risk for developing Diabetes and other pre-diabetic conditions.

US Patent No. 8,476,008 describes methods for diagnosis of pre-diabetes and diabetes using biomarkers identified in a biological fluid, such as saliva. These biomarkers can be identified using proteomic methods, including but not limited to antibody based methods, such as an enzyme-linked immunosorbant assay (ELISA), a radioimmunoassay (RIA), or a lateral flow immunoassay.

Also of interest are PCT Publications WO2010053919 directed to oral biofilms and methods treating and controlling oral periopathogens causing systemic inflammations and controlling oral-originated systemic diseases.

PCT Publication WO0223191A1 describes the use of multiple markers or parameters to assist in the diagnosis or prognosis of a particular condition or event associated with the systemic vasculature. Examples where such diagnoses or prognoses are useful is in assessing healthy and unhealthy animals including human subjects, diagnosing particular conditions or events such as a vascular disease including cardiovascular, stroke, pulmonary, renovascular, cerebrovascular, thrombotic or generalized arterial or venous condition or event or renal or heart failure and in determining the risk of development of such a condition or event in, for example, healthy subjects or subjects to be exposed to certain risks such as inter alia surgery, a course of therapy or vaccination.

WO0223191A1 further describes a method of assessing the parameters associated with a condition or event of the systemic vasculature or assessing a risk of a condition or event occurring, said method comprising obtaining a biological sample from a subject to be tested wherein said biological sample comprises one or more members which are present, absent, elevated or otherwise activated or up or down regulated in a subject prior to, during or following said condition or event and contacting said biological sample with a second set of members wherein one or more of said second set of members are binding partners to one or more of said first set of members and wherein the pattern of interaction between said first and second sets of members including the absence of interaction is indicative of said condition or event or the risk of development of same.

WO0223191A1 further describes a method of treatment, said method comprising assessing the parameters associated with a condition or event associated with the systemic vasculature or assessing a risk of a condition or event occurring, said method comprising contacting a biological sample from a subject to be tested wherein said biological sample comprises one or more members which are present, absent, elevated or otherwise activated in a subject following said condition or event or a condition or event otherwise associated with an aberration wherein said members are selected from two or more of myoglobin, myosin light chain (MLC), myosin heavy chain (MHC), total creatine kinase (CK) including CK-MB, lactate dehydrogenase (LDH-H4), aspartate aminotransferase (AST), cardiac troponin I and T (cTn-I and cTn-T, respectively) and cTn-I and cTn-I RNA, FABP (cardiac fatty acid protein), fatty acid binding protein (FAB protein) including FABP1 and human heart-type, glycogen phosphorylase-BB isoenzyme, α-atrial natriuretic peptide (ANP), cytoplasmic FABP, brain natriuretic peptide (BNP), adrenomedullin (ADM), low density lipoprotein (LDL), very low density lipoprotein (VLDL), high density lipoprotein (HDL) and intermediate density lipoprotein (IDL), C reactive protein (CRP), serum amyloid A, P-selectin, prostaglandins, platelet-activating factor (PAF), histamine, tumor necrosis factor α (TNFα), soluble TNF receptor 2 (sTNFR2), fibrin, fibrinogen, fibrinolytic peptides, modified haemoglobin (HbA1c), ferritin, soluble intercellular adhesion molecule (ICAM) including soluble intercellular adhesion molecule-1 (ICAM1), heat shock proteins, adiponection, Apolipoprotein A (ApoA), Apolipoprotein B (Apo B), apoE, E-selectin, IL- Iα, IL-I β, IL-4, IL-5, IL-6, IL-8, IIIL-Iβ, IL-IO, IL-13, IL-18, B-natriuretic peptide (BNP), NT-proBNP, MCP-I, MPO, Intercellular Adhesion Molecule (ICAM), Vascular Cellular Adhesion Molecule (VCAM), soluble vascular cell adhesion molecule-1 (VCAM1), sICAM-1, myeloperoxidase, CD40L, sCD40L, IFN-γ, ENA78, fractalkline, PIGF, PAPP-A, RANTES, D- dimer, IMA, FFAu, choline, homocysteine or parts thereof, *Streptococcus* sp., *Porphyromonas gingivalis, Helicobacter pylori* and *Chlamydia pneumoniae* or immunological relatives thereof, necrosis and platelet markers, leptin, vasopeptidase inhibitor of cardiac endogenous kinins, heparin, MMP metalloproteinase-9 (MMP-9), metalloproteinase-1 including its tissue inhibitor, angiotensin-converting enzyme, CD95/Apo1/Fas, hepatocyte growth factor, plasma brain natriuretic peptide, angiotensin II type receptor, endothelial constitutive nitric oxide synthase, glycoprotein IIIa genetic polymorphisms, factor Vlla, vWF, thrombin, endothelin-1, cardiac myofibrillar proteins, Fas and Fas ligand, ligands thereof or binding partners thereof or nucleic acid molecules encoding same or their fragments or ligands or binding partners (or a combination thereof) and contacting said biological sample with a second set of members wherein one or more of said second set of members are binding partners to one or more of said first set of members and wherein the pattern of interaction between said first and second sets of members including the absence of interaction is indicative of said condition or event or a condition or event, and then effecting a suitable treatment regimen.

WO0223191A1 further describes an array of binding partners for members in a biological sample from a subject said members being present, absent, elevated or otherwise activated in a subject following a condition or event associated with the systemic vasculature wherein the binding partners are defined by (x,y) coordinates such that the array comprises n binding partners at coordinates (x,y), (x₂, y₂) ... (xₙ,yₙ) and wherein the pattern of interaction between the members and the binding partners is indicative of said condition or event.

WO0223191A1 further describes a method for estimating the size of an infarct or related condition in a subject wherein the size of the infarct (Is) is determined by the formula $Is = \frac{{\int }_{0}^{t} f \left(t\right) dt \times Bw \times Kw}{Ed \times Kr}$
wherein "Is" is infarct size; F(t)dt is the rate of release of a member in a biological sample, said member being present, absent, elevated or otherwise activated in a subject following a cardiovascular condition or event leading to the infarct; [f(t) is also known as the member appearance function]; Bw is the body weight of the subject; Kw is the proportion of the body weight into which the member is released; Ed is the rate of removal of the member from evaluation; and Kr is the total amount of member released divided by the amount of the member released from the infarcted tissue; said method comprising contacting a biological sample from said subject wherein said sample comprises members present, absent, elevated or otherwise activated in a subject following a cardiovascular condition or event or a condition or event otherwise associated with a cardiovascular aberration with one or more binding partners of said members wherein the binding partners are immobilized to a solid support and wherein the pattern of interaction between the members and binding partners is indicative of the size of the infarct or otherwise provides data input for assessment of the size of the infarct.

WO0223191A1 further describes a method of assessing the parameters associated with a condition or event associated with the systemic vascularization, said method comprising screening for the presence of two or more mRNA molecules in a biological sample which mRNA molecules are translatable to members which are present, absent, elevated or otherwise activated following a cardiovascular condition or event or a condition or event otherwise associated with a cardiovascular aberration said screening comprising contacting the biological sample with an array of oligonucleotides capable of hybridizing or otherwise capturing said mRNA molecule and detecting said hybridization or captive and wherein presence or absence of said mRNA molecule is indicative of a said condition or event or a risk of development of same.

WO0223191A1 claims (a) the detection of two or more biomarkers, (b) evaluating biomarker levels with respect to a scoring index, wherein evaluation comprises: (i) assigning an index to each biomarker or combination of biomarkers based on its/their measured capacity to discriminate between cardiac healthy subjects and cardiac disease patients, (ii) establishing a threshold level of the biomarker with an index greater than 0.8 to discriminate cardiac healthy subjects from cardiac disease patients; and (c) determining a value representative of the cardiovascular disease status of the subject based on the evaluation of subject's biomarker. In some embodiments, the detection device is lab-on-a-chip.

PCT Publication WO2013020870 discloses a method for a cardiovascular risk assessment in a subject, or for a reclassification of a subject to an improved risk assessment compared to that obtained using the scales/methods for such risk estimation such as, but not limited to Framingham, Regicor, Score, Procamor Qrisk, comprising the steps of determining in a sample isolated from said subject the presence of certain polymorphisms in particular sequence, indicative of a risk of having a cardiovascular event. In some embodiments, the method determines the probability of an individual of presenting a fatal or non-fatal myocardial infarction or angina in a 10 year period based on the presence of certain classical risk factors and certain polymorphisms using the formula: $1 - {{S}^{⌢}}^{exp \left[{\sum }_{p = 1}^{P} {β}_{{CRF}_{p}}*{CRF}_{p , i}+{\sum }_{j = 1}^{J} {β}_{{SNP}_{j}}*{SNP}_{j ,i}-{\sum }_{p = 1}^{P} {β}_{{CRF}_{p}}*CR \overline{{F}_{P}}-{\sum }_{j = 1}^{J} {β}_{{SNP}_{j}}*\overline{{SNP}_{j}}\right]}$
wherein Ŝ is the mean survival free of coronary events at the population, p
${Σ}_{p = 1}^{p}$ is the summatory function along the P classical risk factors,
${β}_{{CRF}_{p}}$ the logarithm of hazard ratio corresponding to the classical coronary risk factor "p",
CRF_{p,i} is the value of each coronary risk factor "p" included in the equation for an individual "i", 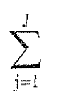
is the summatory function along the J genetic variants.
${β}_{SNP j}$ is the logarithm of hazard ratio corresponding to the genetic variant "j".

SNP_{j,i} is the number of risk alleles (0,1,2) for a specific genetic variant "j" included in the equation for an individual "i".

C̅R̅F̅_̅{̅P̅}̅ is the average value for the classical risk factor "p" in the population.

S̅N̅P̅ⱼ̅ is the average risk allele number of copies for genetic variant "j" in the population.

Other formulas disclosed therein are: $= {β}_{chol} * \left({cholesterol}_{i}-6\right) + {β}_{SPB} * \left({SBP}_{i}-120\right) + {β}_{s m o ker} * {current}_{i} + {{\sum }_{j = 1}^{J} {β}_{{SNP}_{j}}}_{⊥}$

The cardiovascular event can be selected from the group of fatal or non-fatal myocardial infarction, stroke, angina pectoris, transient ischemic attacks, peripheral arterial disease or a combination thereof. Cardiovascular disease or disorder risk factor(s) are selected from the group consisting of age, race, sex, body mass index, blood pressure, smoking status, low density lipoprotein (LDL)- or high density lipoprotein (HDL)-cholesterol level, systolic blood pressure, diastolic blood pressure, history of heart failure, diabetes, renal insufficiency, left ventricular hypertrophy, alcohol consumption history, smoking history, exercise history, diet, and family history of cardiovascular disease or disorder.

Additional US patents may be of interest in employing the present disclosure:
US Patent 9,037,477 describes a computer-implemented method for evaluating ambulatory electrocardiographic (ECG) monitoring of cardiac rhythm disorders, wherein a patient is registered online and medical records for the patient are assembled, and an ambulatory ECG monitor is registered to the patient for retrieval of an electrocardiogram from its recording circuitry. The electrocardiogram and medical records are evaluated against diagnostic criteria. An RFID tag and detachable tracking ticket can be employed in the system, which can interface with a clinician and an RFID transceiverequipped computer or mobile computing device such as a smart telephone, to read from and record into the RFID tag. The computer includes components conventionally generally found in programmable devices, such as a central processing unit, volatile memory, input and output ports, user display, keyboard or other input device, network interface, and non-volatile mass storage. Other components are possible. In some embodiments, the patient electronically transmits the recorded ECG data to a referral center following completion of monitoring. In some embodiments, the patient is given an electronic or traditional paper diary (or instructed how to access a dictation service that makes diary entries for the patient) to help chronicle physical symptoms, subjective feelings, and activities at the time of symptomatic onset during monitoring. A software-implemented diary can be provided in several forms, such as distributed on machine-readable media, or downloaded online, which, when installed on a computing device, locally executes an electronic diary application. The diary software can be a virtual application loaded from a remote server, such as a Web-based application that executes in a Web browser on the patient's computing device. The diary software could be part of an existing software application, like a personal information manager and communications program, such as Outlook, licensed by Microsoft Corporation, or a social networking and microblogging service, such as Twitter, licensed by Twitter, Inc. Preferably once a day or as appropriate, the patient types or dictates entries into the diary. A technology-assisted dictated diary uses an existing electronics infrastructure to accept spoken diary entries from the patient, who is asked to telephone a call monitoring center to dictate his diary entries daily. When dictated, voice recognition software may convert the patient's spoken words into text, or the speech can be manually transcribed into text off-line by a third party transcription service. Test results can be transmitted electronically to a physician. Patients and authorized physicians can access the test results through a secure Web page. Follow up may similarly be electronic, voice or mail.

US Patent 9,020,934 is directed to a computer executable method, an arrangement and a computer program product for analysing a biological or medical sample, including characterizing, e.g. for diagnostic purposes, utilizing a reference database, a query sample tissue based on the gene expression data of the tissue. The method comprises the steps of calculating for the genes of the query sample an expression match score (EM-score) indicating the likelihood of having the gene expression level observed in the query sample in each of the tissue categories of the reference database, calculating for the genes of the sample tissue, using e.g. the EM-score, tissue specificity score (TS-score), that expresses how uniquely a gene identifies the query sample as belonging to a certain tissue category, calculating, utilizing e.g. the TS-score, overall similarity of the sample tissue in relation to a tissue category of the reference database, and storing at least some resulting characterization data to a memory device or outputting the data to an output device of a computer. Further described are building expression level density estimates for each gene of a tissue category of the reference database, wherein the step of calculating the expression match score of a gene of the query sample vis-a-vis a tissue category in a reference database, as well as identifying at least one reference patient based on the identified tissue category, and performing, based on the properties of the at least one reference patient, at least one of the following: i. establishing a diagnosis of the disease, ii. recommending a medication for the disease, and iii. estimating clinical outcomes with a suggested medication.

US Patent 9,013,690 describes a highly sensitive method for detection of biomarkers and/or diagnosis of a disease or condition by obtaining a liquid sample from a patient suspected of having a disease or condition, using a nanosensor comprising a substrate and one or a plurality of pillars extending from a surface of the substrate, where the pillars comprise a metallic dot structure, a metal disc, and a metallic back plane. The nanosensor comprises a molecular adhesion layer that covers at least a part of the metallic dot structure, the metal disc, and/or the metallic back plane and a capture agent bound to the molecular adhesion layer. The nanosensor amplifies and reads a light signal (fluorescence, electroluminescence, chemiluminescence, or other luminescence) from an analyte, when the analyte is specifically bound to the capture agent and said light signal detects and/or quantifies said biomarker in said sample and provides a diagnosis of said disease or condition. The biomarkers can be a protein, nucleic acid, or other molecule or chemical compound. The liquid sample can be amniotic fluid, aqueous humour, vitreous humour, breast milk, cerebrospinal fluid (CSF), cerumen (earwax), chyle, chime, endolymph, perilymph, feces, gastric acid, gastric juice, lymph, mucus, nasal drainage, phlegm), pericardial fluid, peritoneal fluid, pleural fluid, pus, rheum, sebum, semen, sputum, sweat, synovial fluid, tears, vomit, urine or exhaled condensate.

US Patent 9,005,119 describes a Medical Diagnostic and Treatment Advice (MDATA) system and method for providing computerized, knowledge-based medical diagnostic and treatment advice. The medical advice is provided to the general public over networks, such as a telephone network or a computer network. The invention also includes a stand-alone embodiment that may utilize occasional connectivity to a central computer by use of a network, such as the internet. New authoring languages, interactive voice response and speech recognition are used to enable expert and general practitioner knowledge to be encoded for access by the public. "Meta" functions for time-density analysis of a number of factors regarding the number of medical complaints per unit of time are an integral part of the system. A re-enter feature monitors the user's changing condition over time. A symptom severity analysis helps to respond to the changing conditions. System sensitivity factors may be changed at a global level or other levels to adjust the system advice as necessary. The computer system conducts automated interviews of patients for the purpose of establishing a medical diagnosis. The MDATA system consists of a number of databases of medical information and programs (e.g. diseases, symptoms, treatments, medications, scripts) as well as supporting software to provide services to its user community (patients, doctors, nurses, laboratories, health management organizations (HMOs)).

US Patent 9,002,682 describes a method, computer system, and computer memory medium optimizing a transductive model Mx suitable for use in data analysis and predictive systems for determining a prognostic outcome specific to a particular subject. The particular subject may be represented by an input vector, which includes a number of variable features in relation to a scenario of interest. For example, the method can determine the prognostic outcome for particular subject x represented as input vector x, wherein input vector x comprises a number of variable features in relation to a scenario of interest for which there is a global dataset D of samples also having the same variable features relating to the scenario as input vector x, and for which an outcome is known, the method comprising: (A) optimizing the transductive model by: a) determining what number and a subset Vx of variable features of input vector x will be used in assessing an outcome for the input vector x; b) determining what number Kx of samples from within the global data set D will form a neighborhood about input vector x; c) selecting suitable Kx samples from the global data set which have the variable features that most closely accord to the variable features of the particular subject x to form the neighborhood Dx; d) ranking the Vx variable features within the neighborhood Dx in order of importance to the outcome and obtaining a weight vector Wx for all variable features Vx; e) creating a prognostic transductive model Mx for each input vector x, having a set of model parameters Px and the other parameters Vx and Kx from elements a)-d); f) testing an accuracy of the model Mx for each sample from Dx method selected from the group consisting of: (i) calculating Wx as normalized SNR (Signal-to-Noise Ratio) coefficients and sorting the variables in descending order: V1, V2, ... , Vv, where: w1>=w2>= ... >=wy, calculated as follows: w.sub.1=abs(M1.sup.(class 1,x)-M1.sup.(class 2,x))/(Std1.sup.(class1)+Std1.sup.(class2)); (ii) testing for all variables Vx all possible combinations of values of their weights Wx are tested through an exhaustive search to maximize the overall accuracy of a model built on the data Dx; (iii) applying a genetic statistical analysis procedure, if the number of variables prevents using method (ii) above; (iv) applying a quantum inspired evolutionary statistical analysis technique, to select the optimal variable set Vx for every new input vector x and to weigh the variables through a probability wave function; g) storing both the accuracy and the set of model parameters; h) repeating elements a) and/or b) while applying an optimization procedure to optimize Vx and/or Kx, to determine their optimal values, before repeating elements c)-h) until the accuracy is maximized, wherein a number and a subset Vx of variable features of input vector x, and a number Kx of samples from within the global data set D that form a neighborhood about input vector x are determined anew each time elements a) and b) are repeated while applying an optimization procedure to optimize Vx and/or Kx; (B) determining a prognostic outcome y specific to the patient x using the optimized transductive model Mx by: (I) forming a vector: Fx={Vx,Wx,Kx,Dx,Mx,Px,t}, where the variable t represents the time of the model Mx creation; (II) calculating the weighted distance D(Fx,Fd) as an aggregated indication of how much a person's profile should change to reach an average desired profile Fd by using the following: D(Fx,Fd)=.SIGMA..sub.I=1,vabs(V.sub.Ix-V.sub.Id)w.sub.I; (iii) designing a vector of required variable changes, defined as: deltaFx,d=(deltaV.sub.lx,d), for I=1,v as follows: deltaV.sub.lx, d=V.sub.lx-V.sub.ld, with an importance of: WI; (C) modifying variable features Vx in the patient x to be closer to Kx values associated with an improved outcome relative to the prognostic outcome y determined for the patient x so as to improve the prognostic outcome of the patient x; and (D) repeating elements a) through h) to determine an improved prognostic outcome using re-optimized transductive model Mx. In one embodiment, a subset of the variable features within a neighborhood formed by the samples are ranked in order of importance to an outcome. The prognostic transductive model is then created based, at least in part, on the subset, the ranking, and the neighborhood. The subset and the neighborhood are then optimized until the accuracy of the transductive model is maximized.

US Patent 9,002,654 describes a diagnostic method employing multi-analyte analysis of saliva biomarkers as predictors of periodontal and pre-implant disease to determine probability of an oral disease state. The method comprises (a) determining the levels of two or more biomarkers in a sample collected from a first individual, wherein a first biomarker is a bone-specific marker and a second biomarker is a plaque biofilm pathogen marker, said levels of said two or more biomarkers indicating the probability of said oral disease state, wherein the first biomarker is not type I collagen pyridinoline cross-linked telopeptide (ICTP); wherein elevated levels of said two or more biomarkers from said first individual compared to levels of identical biomarkers from a second, healthy individual, or compared to biomarker levels of said first individual measured at an earlier time point are indicative of occurrence of oral disease in said first individual with a probability of diagnosing the disease state equal to or greater than 70%; and (b) treating said oral disease by administering an amount of a therapeutic or prophylactic composition sufficient to reduce activity of said two or more biomarkers. methods of measuring biomarkers to determine the probability of a periodontal and/or peri-implant disease. The biomarkers are selected from the group consisting of *Aggregatibacter actinomycetemcomitans, Campylobacter rectus, Fusobacterium nucleatum, Prevotella intermedia, Porphyromonas gingivalis, Tannerella forsythia, Treponema denticola,* matrix metalloproteinase-8 (MMP-8), matrix metalloproteinase-9 (MMP-9), osteoprotegerin (OPG), interleukin-1 beta (IL-1.beta.), interleukin-6 (IL-6), interleukin-4 (IL-4), interleukin-10 (IL-10), interleukin-2 (IL-2), interleukin-13 (IL-13), calprotectin, tumor necrosis factor .alpha. (TNF.alpha.) and combinations thereof.

US Patent 8,970,381 describes a computer-based system and method for providing coordinated health monitoring, emergency response, and medical record delivery. The system can include computing devices configured to process emergency-related indicators and data, and can also include monitoring devices communicatively linked to the computing devices. The monitoring devices can be configured to monitor a particular area for the emergency-related indicators and data, wherein the monitoring devices detect speech, sounds, images and other detectable emergency-related indicators. The monitoring devices can also be configured to transmit the emergency-related indicators and data to the computing devices. Furthermore, the system can include a module linked to the monitoring devices and configured to execute on the computing devices. The module can analyze the transmitted emergency-related indicators and data to determine whether there is an emergency, communicate with a monitoring service to validate that an emergency exists, and provide access to patient records to authorized personnel, when an emergency exists.

US Patent 8,924,236 describes a system and process for providing a computerized medical and biographical records database and diagnostic information. A medical records database and diagnostic program is stored on a central computer that is accessible to individuals using remotely situated computers connected to a computer network. Individual patient medical and biographical records are owned by individual patients who can enter information in their record as well as grant or deny authorization to others, such as health care professionals, insurance providers and other entities, to review part or all of their record. The method, process and system establishes the rules and parameters of function of providers and users of the services (such as delivering and receiving health care services), stores all available data, provides the functional platforms for all medical, non-medical, and financial transactions to occur in an electronic, softwareguided, anonymous, efficient, and uniform environment.

US Patent 8,918,771 describes a decision tree ensemble compilation. In one embodiment, a decision tree is evaluated in interpreted mode while statistics are collected. The decision tree is then represented as source code, and each decision in the decision tree is annotated with instructions determined based on the collected statistics. The source code is compiled into machine code, and the machine code is optimized based on the instructions annotating each decision in the decision tree. In some embodiments, the decision tree is evaluated multiple times in interpreted mode. In some embodiments, the collected statistics comprise, for each decision in the decision tree having a plurality of possible outcomes, a probability that each possible outcome of the decision is actually realized while the decision tree is evaluated in interpreted mode. In some embodiments, the compiling the source code into the machine code comprises: for each decision in the decision tree, ordering the machine code representing the plurality of possible outcomes of the decision based on the total number of times each possible outcome of the decision is actually realized while the decision tree is evaluated in interpreted mode.

US Patent 8,706,521 describes a method, for use with at least one processor on at least one computer, of generating a report to help decide among a plurality of treatment options for a patient with a given treatable medical condition, the method comprising: receiving patient information related to the patient and the medical condition; querying a treatment option database to generate a plurality of potential treatment options for the medical condition; receiving preference information indicative of the patient's preference for potential treatment outcomes of the treatment options to produce a preference value; analyzing indexed study data relating to the plurality of treatment options by at least grading evidence within the indexed study data, wherein the indexed study data is derived from scientific studies, each of which evaluates an effect of a different treatment option on different trial subjects who have a similar medical condition as the patient; producing a study score for each of the treatment options based on the analysis of the indexed data; determining, for each distinct treatment option, a distinct treatment score as a function of at least the preference value and the study score corresponding to the distinct treatment option; generating a report listing the treatment options and a) the treatment scores or b) information derived from the treatment scores. In some embodiments, the information derived from the treatment scores is a ranking of the treatment options as a function of the treatment scores for the treatment options. In some embodiments, the method further comprises determining that a difference between a highest treatment score and a next highest treatment score is larger than a threshold; determining that no single value used to compute the highest treatment score is greater than the next highest treatment score or the value used to compute the next highest treatment score; and selecting a recommended treatment option with the highest treatment score. In some embodiments, the method further comprises choosing two treatment options with the highest treatment scores; determining if a difference between the two highest treatment scores falls below a pre-defined threshold, and if so, recalculating the treatment scores without using the preference value. In some embodiments, the method further comprises comparing the patient information with characteristics of trial subjects tested in at least one scientific study represented by the indexed study data to determine the applicability of at least one study to the patient, represented by an applicability value; and recalculating, for each treatment option, the treatment score as a function of the preference value, study score, and applicability value. In some embodiments, the method further comprises receiving treatment recommendations from a plurality of experts, the treatment recommendations relating to the plurality of treatment options; converting, at least in part in a computer process, the treatment recommendations into an expert score for each treatment option; and for each treatment option, recalculating the treatment score as a function of the preference value, study score, and expert score. In some embodiments, the method further comprises applying an expert weight to the expert score. In some embodiments, the expert weight is calculated using at least one of a ranking of the expert's academic institution, a ranking of the expert's employing institution, the expert's previous success in recommending, the expert's degree of experience, and the relatedness of the expert's qualifications or experiences to treating or working or processing conditions of the patient. In some embodiments, the method further comprises storing, in an outcome database, treatment decisions by a plurality of patients together with medical outcomes of the treatments and reporting to the patient information derived from the outcome database related to past outcomes. In some embodiments, the method further comprises determining the treatment scores as a function of the success rate in the outcome database. In some embodiments, the method further comprises formulating a medical question, the question comprising patient information, information about the medical condition, and a list of the potential treatment options. In some embodiments, the method further comprises sending the question to a panel of experts, receiving response information from the experts about treatment options and reporting the response information to the patient. In some embodiments, the method further comprises creating the treatment option database. In some embodiments, the method further comprises creating a database with the indexed study data. In some embodiments, each study score also corresponds to an effectiveness demonstrated by the treatment option in a distinct study.

US Patent 8,249,326 describes methods and an apparatus for automated assessment of tissue pathology involve computing an index having a value based upon measures of a plurality of morphological features of cell nuclei in the tissue. The method can include obtaining images of a plurality of cell nuclei in a tissue sample; computing values for a plurality of morphometric features of the plurality of cell nuclei, the morphometric features including one or more texture features; and, from the values of the morphometric features, computing a value for an index characterizing tissue of the tissue sample; and, recording the index value; wherein computing the value for the index is performed according to a function having the property that there exists a range of values for the index for which there is a confidence in excess of 50% that the tissue belongs to a nonprogressing phenotype if the index for the tissue is in the range. The methods may be performed completely automatically or semi-automatically. The index value can be predictive of outcome. The index value may be determined by computing discriminant scores for the cell nuclei based upon values of the measures of morphological features and classifying the nuclei into bins based upon the discriminant score values. The index may be based upon proportions of the nuclei classified in different ones of the bins.

US Patent 7,698,154 describes a patient-controlled automated medical record, diagnosis, and treatment system and method for providing a computerized medical and biographical records database and diagnostic information. A medical records database and diagnostic program is stored on a central computer that is accessible to individuals using remotely situated computers connected to a computer network. Individual patient medical and biographical records are owned by individual patients who can enter information in their record as well as grant or deny authorization to others, such as health care professionals, insurance providers and other entities, to review part or all of their record. The method can include collecting patient health data via sensors connected to remotely situated computers; storing the collected data to memory to form a library of patient data records measured over time; comparing the collected patient health data to the library of patient health data records; and d. correlating any variation between the collected patient health data and the library of patient health data records, the degree of variation between the collected patient health data and the library of patient health data records, responses to the medical diagnostic questions, and the relative weight of the medical diagnostic questions to potential diagnoses. The diagnostic program provides a series of diagnostic questions (relating to medical signs and symptoms) to an individual who must respond either "yes" or "no" to each question. Each potential response is weighted relative to its importance to a particular disease diagnosis. Relative weights for all responses to diagnostic questions are summed to identify potential diagnoses to connected to the answered questions. The diagnostic program provides the individual with a list of potential diagnoses as well as permitting the individual to save the information to his or her individual medical and biographical record. The information maintained in the above system and process is used for health care financing and insurance.

US Patent 6,736,776 describes a method for diagnosing and interpreting dental conditions using a computer system. An image of the lesion being diagnosed is captured and terms describing the lesion are selected. A differential diagnosis list of the most probable lesions is returned. The user views details about each listed lesion until a match is selected, and appropriate medications for the selected lesion are presented. Medication details are reviewed and a proper medication to prescribe is selected. The user can generate a prescription, treatment algorithm, directions report, or a medication report. If the user is uncomfortable with the diagnosis, a referral report can be generated. For performing routine interpretation of dental conditions, the user captures an image for digital x-ray analysis. The user selects the task, such as caries detection, for which to optimize the image. The system optimizes the image based on the task selected and displays the optimized image.

US Patent 5,978,466 describes an anonymous testing system for taking a sample of body fluid to be tested, wherein the sample is acquired in private and sent for analyzation to obtain results. The system comprises a test kit for creating a sample of body fluid, a personal code for anonymously identifying the sample and the person, and an electronic file telephonically created and accessed by the person taking the test and identified by the personal code. The electronic file contains the test results determined by analyzation. There is also a method of anonymously testing of a person's body fluid in private and the results to be anonymously received by the person without having to reveal his or her identification. The method comprises the steps of procuring a test kit for taking a sample of the body fluid. The test kit has a personal code associated therewith and equipment to take the sample. The person uses the equipment to obtain the sample and sends the sample with the personal code to a testing site. The person also creates a personal electronic file telephonically; the file is identified by the personal code. The sample is identified by the personal code. At the testing site, the sample is tested, and the results are updated into the electronic file with a corresponding personal code. The person later accesses the electronic file telephonically to obtain his or her results.

US Patent 5,714,341 describes an improved method for determining the presence of an analyte in an oral fluid sample. A portion of the sample is mixed or contacted with a chromogenic substrate effective to produce a colored product upon reaction with alpha-amylase present in the sample. After a selected reaction time, the reaction mixture is inspected to determine the level of such colored product produced. Production of a level of colored product above a selected threshold within the reaction time indicates that an effective volume of undiluted oral fluid sample has been collected which is sufficient to allow detection of the analyte. A device for use in the method is also disclosed.

Also of interest is US patent application publication 20150066172 and PCT publication WO 2015/031127, directed to an activity tracking device having a display screen, a data store, a main processor communicatively coupled with the data store, a device profile configuration stored in the data store, the device profile configuration comprising a device identifier and a plurality of processor identifiers; and a plurality of device statuses; a user profile configuration stored in the data store, the user profile configuration comprising: a user identifier; and a minimum user activity parameter. The minimum user activity parameter is determined based on one or more historical activity parameters resulting from actual user activity as tracked by the activity tracking device, stored in the data store, and configured in the user profile configuration. In some embodiments, the user profile configuration further comprises an indicator of user selection of a messaging library, selected from a plurality of messaging libraries. The plurality of messaging libraries comprises a graphical messaging library, humorous messaging library, a non-humorous messaging library, a motivating messaging library, a themed messaging library, informative messaging library, coaching messaging library, or combinations thereof. In some embodiments, the display screen is configured for display of one or more of total number of steps taken by a user, activity parameter, total number of calories burned, total distance travelled by a user, progress indicator toward user daily steps goal, physical activity intensity indicator, synchronization progress indicator, or a clock with a current time. In some embodiments, the display screen is configured for display of one or more scenarios based on at least one rule, the one or more scenarios comprising a challenge between users, battery settings, encouraging a user based on the actual user activity, encouraging a user based on the activity tracking device not being recently worn, activation of the activity tracking device, morning wake up, upload reminder, achieving goals, firmware update, or combinations thereof. In some embodiments, the one or more historical activity parameters resulting from the actual user activity are calculated by dividing a total number of steps recorded by a total time duration during which the steps were recorded. In some embodiments, the device further comprises a pedometer. In some embodiments, the device further comprises environmental information stored in the data store, the environmental information based on an actual real-time user environment. In some embodiments, the device further comprises environmental information stored in the data store, the environmental information based on a predicted user environment at a future time when an activity is scheduled. In some embodiments, the device is configured for wireless communication or wired communication with a computing device or an additional device. In some embodiments, the wireless communication comprises one or more of radio frequency communications such as cellular, IEEE 802.11 format, IEEE 802.15.1 format, IEEE 802.15 format, or Bluetooth^{™} low energy wireless formats. Also disclosed is a method of initiating an activity challenge between two or more users, the method comprising: a first activity tracking device having a first user profile configuration stored in a data store; a second activity tracking device having a second user profile configuration stored in a data store; setting the first activity tracking device to a challenge mode; bumping the first activity tracking device and the second activity tracking device against each other and causing an electronic wireless communication to occur between the first activity tracking device and the second activity tracking device, wherein the electronic wireless communication initiates the activity challenge between the first activity tracking device and the second activity tracking device.

Any pertinent data (current status and test results as well as medical and dental histories) gathered from any one or more of the herein disclosed devices / programs / methods can be incorporated and used in the presently disclosed method and system.

None of these illustrative publications describe the presently disclosed and claimed subject matter directed to systems and methods of assessing / evaluating wellness status, suggesting considerations, calculating risks and designing a global wellness treatment / maintenance plan and providing additional ideas and recommendations for nextlevel care based on input from dentistry, medicine, physiology, biochemistry and genetics.

In contrast, the present disclosure provides a system for assessing global wellness and developing a maintenance / treatment program for a subject, said system comprising: (i) a computer-readable storage medium comprising a recorded medical and dental history of the subject; (ii) a sample-collection apparatus for obtaining an oral sample from the subject, comprising a plurality of analyte detection regions in direct or indirect fluid communication with said sample-collection apparatus, wherein the analyte detection regions test for the presence of (a) nucleic acids from bacterial or pathogens; and (b) endogenous genetic biomarkers of inflammation and infection; and wherein the analyte detection regions further provide a measure of levels of specific hormones or lipids; and (iii) a computer processor for assessing data from the analyte detection regions of (ii) and the recorded medical and dental history of (i), wherein the processor computes and provides an output recommendation for a maintenance / treatment program.

The present disclosure also provides a method for assessing global wellness and developing a maintenance / treatment plan for a subject, comprising: (i) compiling medical and dental history data points from a subject into a system comprising a computer-readable form; (ii) obtaining an initial periodontal status and a biological sample (e.g., blood, oral or saliva) from the subject; (iii) comparing the medical and dental history data points and the initial periodontal status to a reference set of desired health and wellness goals; (iv) comparing the periodontal status, medical and dental history data points to the reference set of desired restored health and wellness goals; (v) computing a path (a series of behavioral steps) that moves the patient toward restored health and wellness from the initial periodontal status, medical and dental history data points, through optional intermediate stepped results, to the reference set of desired health and wellness goals according to a minimum step criterion; and (vi) determining a treatment plan to adjust the subject's behaviors from the initial periodontal status, medical and dental history data points to the reference set of desired health and wellness goals. In some embodiments, the method further comprises persistently storing the subject's actual achieved intermediate stepped results as a function of time. In some embodiments, the method further comprises re-computing a path to restored health and wellness and altering the treatment plan based on the actual achieved intermediate stepped results as a function of time.

Systems and Methods for Design of a Wellness Treatment! Maintenance Plan (The section headings used herein are for organizational purposes only and are not to be construed as limiting the subject matter described in any way.)

The present teachings are directed to systems and methods of assessing wellness status and designing a global wellness program / treatment and/or maintenance plan for a subject based on input from dentistry, medicine, physiology, biochemistry and genetics.

### A. Compilation of a Subject's Medical and Dental data points

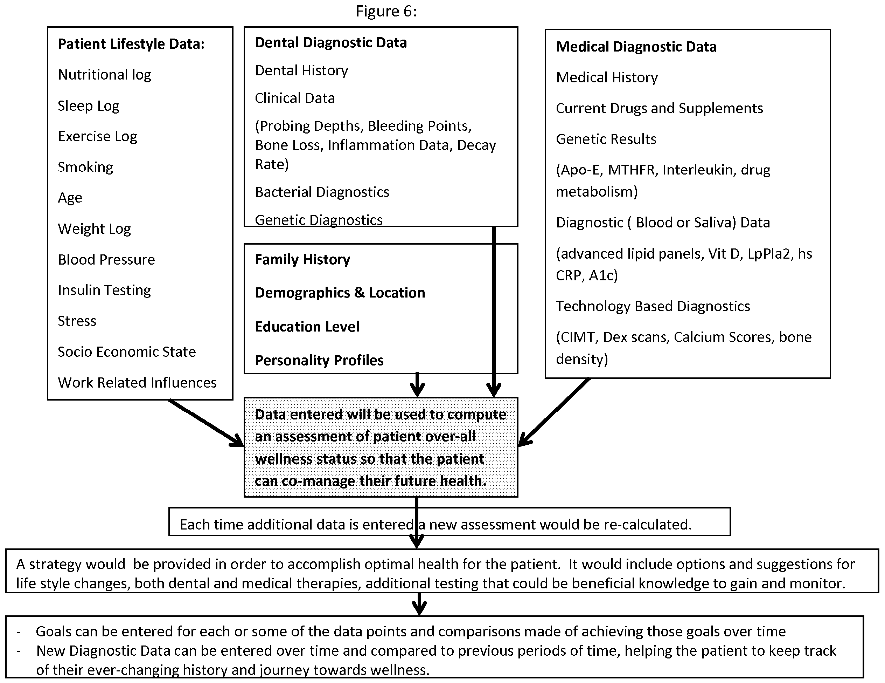

Exemplary data points can include sampling oral nucleic acids and/or proteins for the presence of pathogens associated with periodontal disease [see Figure 6]; Interleukin 1 and 6 genetic testing for inflammatory markers; Apo-E genetic testing for cholesterol; CIMT; advanced lipid testing; LpPlac2 scores; Hs CRP, A1c levels; use of Perioprotect trays; antibiotic therapy associated with periodontal therapy/scaling and root planning; hormone testing or treatment and effects on the oral cavity or cardiovascular disease.

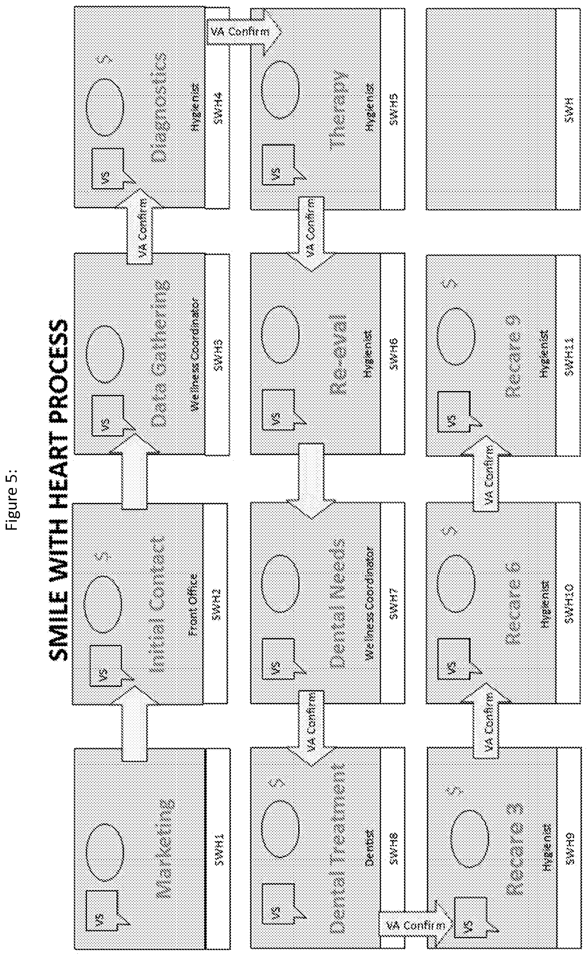

Exemplary Assessment Appointment can include:
Medical and Dental Approach to Prevention and Wellness:
Review & Identify Risk Factors:
Medical History
Family History
Dental History
Symptoms
Previous Diagnostic Testing Results
Existing Prescriptions
Medical Diagnostics
Clinical Signs / Observations
X-Rays & Scans
Diagnostic Testing included but not limited to:
   Advanced Lipid Panels
   Chronic Inflammatory Markers
   Genetic Testing
   Drug Metabolism Testing
   Cancer Testing
   CIMT/Stress Tests
   Body Composition
   Hormone Level
   Nutritional Scores/Deficiencies
   Neutrogenomics
   Sleep studies
   Medical Therapy to Reduce Chronic Inflammation
   Lifestyle Changes:
      Nutrition/Weight Lose/Body Composition
      Supplementation
      Exercise/Skeletal Enhancement/Chiropractic/physical therapy/occupational therapy
      Blood Pressure/CVD
      Hormone Balancing
      Effective Prescriptions for preventive approach to CVD and diabetes specifically but not limited
      Effective prescriptions due to metabolism
      Neutrogenomic Therapy
      Sleep Disorder Therapy
      Dental Diagnostics
      Probing Depths
      Intraoral camera
      X-Rays
      CT Scans (e.g., Dental Cone Beam scans)
      Clinical Signs / Observations
      Bleeding
      Pathogen Testing (e.g., Oral DNA, RNA, protein analysis)
      Genetic Testing
      Cancer Testing
      Blood Diagnostics (fasting and/or non-fasting)
      Nutritional Scores/Deficiencies
      Neutrogenomics
      Sleep studies
      Dental Therapy to Reduce Chronic Inflammation
      Dental Periodontal Therapy in order to reduce the bacterial burden which will influence the reduction of systemic chronic inflammation could include:
         Physical scaling and root planning
         Systemic Antibiotics,
         Local Antibiotic Placement
         Antimicrobial rinses
         Therapeutic trays
         Laser Therapy
         Supplementation
         Maintenance Therapy and frequency based on:
            genetic risk factors, family history, current medical health, genetic results, and comparing future diagnostic blood work, alongside patient's symptoms
         Maintenance Therapy and frequency based on:
            genetic risk factors, family history, current medical health, and comparing future diagnostic pathogen testing and chronic inflammatory markers , clinical signs and etc.
         Lifestyle Changes Knowledge Base and Referral
         Nutrition/Weight Lose/Supplementation
         Hormone Balancing/Endocrinology
         Genetics
         Effective prescriptions for preventive approach to CVD and diabetes
         Effective prescriptions of drug metabolism results
         Neutrogenomic Therapy
         Sleep Disorder Therapy
         Acid Reflux/ENT airway issues
         Patient Education
         Patient Interactive Tracker/ App/Software
         Future advancement in science brought in to approach
         [FIG. 5]: SMILE WITH HEART CLIENT EXPERIENCE
         SWH 1 MARKETING
         SWH 2 INITIAL CONTACT
            - Establish Referral Source
            - Confirm their existing motive for calling
            - Give synopsis of smile with heart program appointment
            - Gather personal data
            - Utilize website or educational pieces to explain program
            - Give Fee or Fee range
            - Awareness of 2 hour appointment
            - Credit card for reserving their time
            - Ask them to send current diagnostics (blood work, CIMT, ORAL DNA from doctor)
            - Fill out health history and return prior to initial appointment
            - Make appointment for diagnostic initial appointment
            - Earliest availability unless the patient has been on recent antibiotics
            - Confirm last antibiotic use and what it was appoint 6 weeks after last dose
         SWH 3 DATA GATHERING
            - Wellness Coordinator will help gather and compile data prior to initial appointment including insurance verification has been done
            - Follow up with doctor's office or patient if data has not been received
            - Review health history and family history and personal info to pass off to therapist and doctor
         SWH 4 INITIAL APPOINTMENT ~2 hours
         Multi Code: NPWELL
            - Build relationship
            - Review health, family, and dental history
            - BP
            - Xrays or scan
            - Chart existing dental Conditions
            - Diagnodent
            - Periodontal charting and tissue provocation
            - BioPhotonic Scan
            - Oral DNA sample
            - oral cancer screen (e.g., Velscope, Oral ID, etc.)
            - Intraoral photos
            - Diagnostic Blood Work (Heart Smart) if available; test levels of A1c, hsCRP, LpPlAC
            - If current health history dictates call in antibiotics and have client start them before the therapy appointment
            - Impressions or scan for perio protect trays (if needed for therapeutic trays)
            - Have client start LifePak Nano and Probiotic if they would like a recommendation for long term use

Without limitation, available tools and screens may be employed in the present method and system. For example, a velscope is a light that, when shown in the mouth can help to visualize oral cancer. There are other screens for oral cancers, such as Oral ID. Early detection is key in successful treatment. Oraquix is numbing gel that can be placed around the tooth to create more comfort on a deep cleaning around a tooth.

As will become apparent from the description herein disclosed, an intervention program / treatment plan designed in accord with the present system and method can correct or even prevent disease rather than merely ameliorate symptoms. Several embodiments of the present disclosure are described in detail hereinafter. These embodiments can take many different forms and should not be construed as limited to those embodiments explicitly set forth herein. Rather, these embodiments are provided so that this disclosure will be thorough and complete, and will fully convey the scope of the present disclosure to those skilled in the art.

The presently disclosed methods and systems for design of a global wellness maintenance or treatment plan is based, at least in part, on an understanding of medicine and dentistry. Examples of medical and dental data points, and their role in the presently disclosed systems and methods for restoration of global wellness will be detailed below.

While these cases represent a spectrum of presentations possible in an initial global wellness assessment, and they may appear to be vastly different in nature, they often have common causality in the sequence of events. Restoring wellness and preventing or reducing risk of future disease, then is the overall goal.

### B. Method and System for Design of a Maintenance or Treatment plan

Provided herein is a method to evaluate and assess a subject's global wellness landscape and to design an intervention and treatment plan that restores proper or desired level of health and wellness. A system for design of a program of an intervention and/or treatment plan in accord with the method is also provided. The method and the system are now to be described with reference to the included Figures.

In the method, a subject experiencing less than ideal wellness or a particular bothersome symptom of ill health is assessed. The subject's initial digital wellness landscape is obtained, and this initial digital wellness landscape is analyzed to evaluate a relationship between the initial digital wellness landscape and a more ideal digital wellness landscape. Based on the analysis, one or more interventions/activities/behaviors to move/change a subject's initial, less-than-ideal data point toward a more ideal data point are identified to restore a patient to a desired or more-desirable state of global wellness.

In one embodiment, the system for application of the method is comprised of a display in the system, which is capable of displaying a digital wellness landscape. A computer program in the system analyzes the digital wellness landscape and evaluates a relationship between the initial digital wellness landscape and an ideal digital wellness landscape, and determines and recommends an adjustment that the subject can make (in diet, behavior, exercise, medications, dental work) to achieve a more ideal state of global wellness. One example of an exercise and fitness routine and equipment system which can be incorporated into the presently disclosed method and system is the "Vasper" fitness system, which bypasses the physical discomfort and physical damage and recovery time required to actually perform intense exercise, and claims to be the most comprehensive full biological systems-conditioning program currently available. Vasper can be used not only for fitness, but also for rehabilitation and recovery from injury or disease.

The presently disclosed system has robust measurement, analytical, assessment and adjustment capabilities for designing and optionally making customized maintenance / treatment plans for each individual subject. The system has the capabilities to compare a subject's digital wellness landscape to a stored ideal digital wellness landscape and/or to measure minimum movements between data points, and additionally has the ability to generate a recommended course of action to achieve an ideal digital wellness landscape. Such a digital wellness landscape includes: (1) data points from medical and dental histories as well as the subject's family history, (2) a computer generated three-dimensional representation of the digital wellness landscape, (3) the ability to determine which genes have been mutated from their wildtype versions, (4) the ability to assess the subject's risk of developing CVD and/or diabetes, and (5) the ability to predict and output a recommended course of action / behavior / treatment / medication. The output medium can be in the form a CD or DVD, or other portable computer-readable form such as a flash drive, for example, or can be in the form of volatile or transmission media. A computer software program in the system analyzes the digital wellness landscape and, based on the analyses, directs the system or a skilled clinician, dentist or physician to prescribe an individualized intervention/ course of action / behavior / surgical or non-surgical treatment / medication, with optional input from the patient/subject, clinician, dentist or physician. The digital wellness landscape, referred to as a restored wellness state, is obtained, and the digital data file of both the initial and the restored wellness states are stored, for use in subsequent evaluation, and additional recommendations as necessary to achieve an ideal state of global wellness.

In use, the computer and its software can be used by a clinician, dentist or physician to obtain a digital wellness landscape. In one embodiment, design of the treatment plan and the software for obtaining the initial digital wellness landscape, and analysis of the digital wellness landscape to ascertain a restored wellness state, has features and capabilities now to be described.

It will be appreciated that adjustments to data points in the subject's medical and dental history and family history can be made by the patient/subject, clinician, particularly if the subject is experiencing any new symptoms / conditions, or a desired state of wellness (e.g., weight loss, reduction of medications and the like). In cases requiring a correction to data points that are too large to achieve the desired global wellness state in a single treatment plan, a series of two or more intermediate treatment plans are designed where the computer defines the treatment plans to progressively move from an intermediate wellness state to the restored wellness state.

Characteristics of the presently disclosed software are: (i) it enables data gathering from patients, physicians, dentists and oral hygienists; (ii) it provides viewing of an initial digital wellness landscape; (iii) it contains rules for displaying the digital wellness landscape in two- and three-dimensional representations, for example, as a vector map allowing visualization of the movement / change from the initial wellness state through adjustments / interventions / behavioral modifications to an improved state of wellness; (iv) it contains rules for assessing current disease states and/or risks of / predispositions to disease, and can suggest courses of action to move toward an improved state of wellness; (v) it contains rules for restoring health, and automatically detecting and updating the recommended adjustments / interventions / behavioral modifications and redrawing lines (for example, to superimpose initial and ideal states of wellness, optionally based on a minimum movements criterion; (vi) it contains rules for determining, at the assessment phase, when serial adjustments / serial interventions / a series of ongoing, stepwise behavioral modifications are needed, and recording the corresponding distances from initial untreated wellness state to the desired and/or prescribed restored wellness state, and computing and/or dividing those distances into intermediate segments/steps for determining a series of intermediate adjustments / interventions / behavioral modifications needed and designed for progressive remediation or achievement of ideal wellness; (vii) it transfers the data for the restored state of global wellness to a clinician or physician's office or to a patient's computer or other user interface; (viii) it will determine how much correction can be performed comfortably for each subject based on information gathered from the assessment mode; (ix) it is able to quantify the relationship between the initial and ideal wellness states and quantify a treatment course / path (i.e., a series of behavioral steps) in order to give practitioners guidance and enhance individual subject treatment / intervention; and/or (x) it can provide a quantitative digital display of the digital wellness states.

### Analysis and Prediction of Prognosis

The computer program of the presently described system performs the above analysis and suggests a course of action / treatment plan for each subject. The program then prescribes treatment options for practitioners who wish to provide additional medical and dental treatment services for in-office correction of health problems, as well as for those who wish to have ongoing progress and re-assessment checkups over the course of a treatment plan.

### Restoration of Wellness Based on the Analysis and Prediction of Prognosis

To restore the subject to a restored wellness state, the system described herein identifies the wide variety of input data points described above, forms an initial digital wellness landscape and then compares this initial digital wellness landscape to a stored ideal digital wellness landscape, then calculates, *in silico*, a plan / path (i.e., a series of behavioral steps that move the subject toward a state of restored wellness, with optional manual input from a clinician, physician or dentist, to bring the two states into congruency.

SWH 5 THERAPY APPOINTMENT (two weeks after initial appointment for 2 hours) Multi Code: PERIO THER
- Review Oral DNA report
- Review diagnostic blood work (Heart Smart) if available
- Therapy (full mouth SC/RP)
- Deliver Sonicare
- Deliver perio protect trays (if prescribed for therapy)
- Rx for antibiotics
- Dental Pearls
- Chlorhexidine rinse
- Instructions
   **The two weeks is to allow for return of results, and if they arrive sooner they could be brought forward, but also consider if anybody needs to meet their stop date of smoking or tobacco use.

SWH 6 SUPPORTIVE PERIODONTAL THERAPY or RE-EVAL (4910 two weeks after Tx) Multi Code: SWH2WEEK & SWH2PRO
- Intra oral photos
- Periodontal charting
- Disclosing and co-therapy instruction
- Therapy (full mouth cleaning) & polish
- Fluoride treatment
- Scan or impress for perio protect maintenance trays (unless restorative is needed first)
- Deliver Air Flosser
- Educate how to utilize the perio protect trays and deliver gel x 3 tubes

**Address urgent restorative needs now. Or possibly on occasion even prior to therapy (ex: extracting)
**Client pick up maintenance perio protect trays or we can mail. Code for delivery must be complete by team member upon delivery.

SWH 7 DENTAL NEEDS PRESENTATION
- Wellness Coordinator or Front office team to review the needed treatment in sequence and costs
- Financial arrangements
- Make the appointment

SWH 8 DENTAL TREATMENT
- Treatment Plan followed by dentist
- impress or scan for perio protect trays after restorative work if completed

SWH 9 RECARE 3 (90 days after SPT)
Multi Code: SWH3MO & SWH3PRO
- Oral DNA retest (Pathogen Only)
- Intra oral photos
- Periodontal charting
- Disclosing and co-therapy instruction
- BioPhotonic scan
- Heart Smart retest if available
- Therapy (full mouth SC/RP)
- Establish future recare intervals
- One CT4 paste
- One CT3 rinse
- Perio protect gels x3

If the client still has infection, one or more of these factors will be considered. Wellness coordinator to review test results if pathogen range is over threshold.
- Recare interval (6 weeks, 3 months)
- Possibly more site specific periodontal therapy/laser therapy
- Changing perio protect tray protocol to therapeutic time and frequency
- Other health issues that may need addressing

SWH 10 RECARE 6
Multi Code: SWH6MO
- Intra oral photos
- Periodontal charting
- Disclosing and co-therapy instruction
- BioPhotonic scan
- Therapy (full mouth SC/RP)
- Establish future recare intervals
- One CT4 paste
- One CT3 rinse
- Three perio protect gels
- Sonicare heads (1 box =2)

SWH 11 RECARE 9
Multi Code: SWH9MO
- Intra oral photos
- Periodontal charting
- Disclosing and co-therapy instruction
- BioPhotonic scan
- Therapy (full mouth SC/RP)
- Establish future recare intervals
- One CT4 paste
- One CT3 rinse
- Three perio protect gels

RENEW PROGRAM:
- Team approach
- Trainers of Trainers vs Traditional Consulting
- Videos provide information economically
- Comfort of doctor's office
- Time convenient to clinician's schedule
- Less down time during hours of operation

EDUCATIONAL TRAINING VIDEOS (proprietary and/or online education programs or platforms, e.g. "Centric Learning," an interactive, online learning platform that houses information that a user can access and be tested on).

### DENTAL WELLNESS MODULES:

- The Relevance to the Oral Systemic Connections
- The Mouth is the Window to Your Health
- Is Your Mouth on Fire?
- Address the Stress
- You Are What You Eat
- The Neurons are the Highway
- The Paradigm Shift to CAMBRA
- Oral DNA Testing Bridges the GAP
- Anti-Aging Techniques can Optimize Your SMILE
- What Does Your Smile Say?
- Sweet Dreams About Sleep
- The New Value Added to Periodontal Therapy and the Therapist!

### BENEFITS OF ONLINE TRAINING:

- Each module video is less than 1 hour
- Easily incorporated into staff meeting
- Self-paced learning
- Learn as a team in the comfort of office
- No travel expenses for team
- Easy to train new team members

### RENEW PROGRAM

- 1 Hour Launch Webinar
- 1 Day Interactive Team Seminar
- Relevant Guide to the Oral Systemic Links
- 12 Educational Training Videos
- Wellness Wednesday Webinars
- Quarterly Calls

### III. Examples

Aspects of the present teachings may be further understood in light of the following examples, which should not be construed as limiting the scope of the present teachings in any way.

### EXAMPLE 1

### Capture of a subject's initial digital wellness landscape:

Subject #1: Patient is a white female, age 42, with two children. She is single, owns her own business, lives in Texas, and has a high level of stress in her life. Financial Bracket 200,000; Education: Post Doctorate; Nutritional Log: Poor; Sleep Log: Poor; Exercise Log: Poor; Weight/ BMI: Obese; Blood pressure Log: Normal; Non-Smoker; Personality Profile (Disc s/c).

Family History: Dad: Breast Cancer, Skin Cancer, Gallbladder and appendix removal, BP High, has lost several teeth. Mother: no history. Siblings: Hodgkins Lymphoma, loss of 1 tooth. Paternal Grandfather: Alzheimers, healthy physically. Paternal Grandmother: High BP, Died of CVD, periodontal disease-dentures. Maternal Grandmother: High BP, Restless leg syndrome, Died of stroke. Maternal Grandfather: Died of Hodgkins Lymphoma.
Dental Diagnostics:
   Probing depths: 2 depths of +4
   Bleeding: 6 sites
   Root canals: 1
   Interleukin 1 :+
   Interleukin 6: ++
Pathogens:
   High Risk pathogens ++
   Medium Risk pathogens +
Clinical Inflammation: Slight
Blood Pressure: Normal
Oxidative stress scan: Low ++
Bone Level: Normal
Dental History: TMJ pain: +
Medical Diagnostics:
   Advanced Lipid Testing
   High Cholesterol: +
   Lp Plac score: +
   hsCRP: +
   Vit D: low : +
Hormones:
   Estrogen : low +
   Progesterone: Normal
   Testosterone: Normal
   Cortisol: low am +
Genetic:
   Apo-E : ¾
   MTHFR : ++
CIMT:
   Plaque: soft +
   Inflammation of wall: ++
Bone Density: Age normal
Symptoms:
   Fatigue: +++
   Stress: ++
General Mood: +
   Foggy Headed Thinking: +
Wake up: Sleepy +
Waist Circumference: +
Neck Circumference: normal
Medical History:
   Pre-eclampsia on pregnancy : +
   Passed out upon workout last year: +
   Adrenal Fatigue in past: +
   Episodes of mild depression
   Prone to fever blisters
   Shingles 3 times
Previous Rx:
   Birth control
   Progesterone creams

### EXAMPLE 2

### Analysis / Assessment of Subject #1

While Subject #1's actual age was 42, her assessed age was 62.

Lifestyle indicates a state of un-healthy: High Risk Factor for chronic inflammatory disease process.

Interventions: Subject #1 advised to educate herself on how the following lifestyle habits contribute to disease: Sleep patterns, Exercise, Weight, Stress.

### FAMILY HISTORY

Family history reveals potential genetic tendencies for:
Cancer
Cardiovascular disease
Periodontal Disease
Alzheimer's

### DENTAL ASSESSMENT

Indicates high risk for early stage periodontal disease.

### Suggestions:

Periodontal therapy with systemic antibiotics
PerioProtect maintenance trays used daily to reduce risks due to genetic inflammatory tendency.
3 month recall

### MEDICAL ASSESSMENT

Indicates a high risk factor for Cardiovascular disease
Alzheimer's

### Suggestions:

Genetically an APO-E ¾. Subject is prone to cholesterol formation from certain foods, and Alzheimer's.
Elevated inflammatory markers suggest to treat periodontal disease, and to have a sleep study for sleep apnea. This could also be contributing to the CIMT inflammation in walls, fatigue, and depression due to the systemic links.

### Suggestions:

Eat accordingly for Apo-E genotype
Weight loss goal of 35 pounds
Increase exercise average 10,000 steps a day to help with insulin resistance, metabolic syndrome
Vaccine for shingles
Monitor blood pressure daily
5,000 IUs daily of Vitamin D
Multi Vitamin Daily
Add B12 shots weekly
Omegas sparingly due to Apo-e 3/4
Start on Estrogen and monitor every three months
Start Pro diam 2 times a day due to positive genetic MTHFR result
Sleep Study for sleep apnea
Stress reduction
Retest CIMT in 1 year
Retest Advanced lipid panel, vitamin D, inflammatory markers in 6 months
Retest hormones in 3 months
Mammogram due to father breast cancer history

### Consider:

Trainer for exercise
Nutritional Chef
Yoga

### EXAMPLE 3

Capturing a subject's initial digital wellness landscape and calculate a path of behavioral steps to move the subject toward a state of restored wellness.

The presently disclosed system and method includes the steps of
(i) compiling medical and dental history data from a subject into a database stored in a computer-readable form;
(ii) obtaining an initial periodontal status from an oral sample from the subject; then, subsequently,
(iii) comparing the medical and dental history data and the initial periodontal status to a reference set of desired health and wellness goals for the subject;
(iv) computing a path of behavioral steps for the patient to arrive at restored health and wellness from the initial periodontal status and medical and dental history data, said path having optional intermediate stepped results, wherein said path ends at the reference set of desired health and wellness goals according to a minimum step criterion; and
(v) prescribing a treatment plan for the subject, guided by the computed path.

In some embodiments, the method may further comprise repeating one or more of steps (i) - (v) at various timepoints over the course of the assessment and treatment.

In some embodiments, the method may further comprise recording the subject's achieved intermediate stepped results as a function of time.

In some embodiments, the method may further comprise re-computing a path to restored health and wellness and altering the treatment plan based on the subject's achieved intermediate stepped results.

A system for assessing global wellness and developing a treatment plan for a subject, said system comprising:
(i) a computer-readable storage medium comprising a recorded medical and dental history of the subject;
(ii) a sample-collection apparatus for obtaining an oral sample from the subject, comprising a plurality of analyte detection regions in direct or indirect fluid communication with said sample-collection apparatus, wherein the analyte detection regions test for the presence of
   (a) nucleic acids from bacterial or pathogens; and
   (b) endogenous genetic biomarkers of inflammation and infection;
   and wherein the analyte detection regions optionally provide a measure of levels of specific hormones or lipids; and
(iii) a computer processor for assessing data from the analyte detection regions of (ii) and the recorded medical and dental history of (i), wherein the processor computes and provides an output recommendation for a treatment program.

Throughout the present disclosure, the words "subject," "user" and "patient" may be used interchangeably. It is also understood that the user may be acting as a proxy for the patient. In such a case, the user is registered as an assistant for the patient.

In a non-limiting example using the presently disclosed system and method, the user may enter current, real time weight and height data, medical testing results (e.g., blood work, genetics), current medications (prescription and/or over-the-counter) and their respective amounts and dosages, current dental status and conditions, current oral care products and habits (e.g., kinds of toothpaste and/or rinses, frequency of brushing, flossing, use of air flosser, use of perio protect trays, etc.), current use of vitamin and minerals supplements and probiotics, current habits (smoking packs per day, drug use, alcohol drinks per week and what kind), and/or family history into the user's computer or smartphone. Additional information about the subject/patient/user, such as general activity level can be gathered. Exemplary genetic markers can include, but are not limited to KIF6, apo-E, IL-1, IL-6, toll-like receptors, tumor necrosis factors (TNFs) and receptors, 5, 10-methylenetetrahydro folate reductase (MTHFR).

As a simple example, in some embodiments, an initial, current BMI and/or body composition (weight and the % of body fat compared to muscle) is calculated, and then a target BMI is prescribed automatically. The presently described system can then determines caloric intake per day necessary to maintain the user's current weight and BMI, and can calculate various rates of weight loss and decrease in BMI (e.g., one pound per week loss, two pounds per week loss, etc.) and prescribe the necessary maximum caloric intake required to lose weight and BMI at that rate. The user can input specific foods and meals eaten, as well as the expenditure of calories when various kinds of cardiovascular, isotonic or isometric exercises, or strength / endurance training, and the system can automatically re-calculate the an equal amount of calories to match the amount expended in exercise to maintain the same rate of weight loss and/or decrease in BMI.

Subjects / patients may also be interviewed in person or over the telephone by a clinician, or via a computer interface. The computer on which the system resides and runs can be a single computer such as a server or a linked system of computers. It can also be configured as several servers connected by a Local Area Network (LAN) or a Wide-Area Network (WAN), in several LANs or WANs that are distributed around the country, or as some combination of these methods. Whichever configuration is used, the computer is the central corporate tool to: develop, install, maintain, update software, store and maintain all system support databases, support medical and dental software research and development, verify, validate, and test the system, document the use of programs and data, support access by patients for assessment and advice, generally manage and control system operation.

In the system and method described herein, hardware and system software are assembled with two basic concepts in mind: portability to other operating systems and the use of industry standard components. Thus, the system is flexible and will allow continual improvements/upgrades to the system, as well as decreasing costs. While specific hardware and software may be referenced, it will be understood that many different components could be used in the present system.

The present system and method may use "scripts", which are, essentially, program modules that know how to interview a patient about a given complaint, access patient medical and dental records, and use the supporting databases to assess and advise patients. The presently described system may comprise a telephone network to connect patients to a computer on which the system runs. In some embodiments, a network may be employed to use communications networks such as the internet to connect the system's computer(s) to an on-line patient using a network access processor or patient computer.

The software is modified or extended to accommodate the internet's communication protocols and the patient computer's storage, processing, and display capabilities. These changes to the system consist of changes in its input/output subsystem, which is modified to communicate with the internet and (via the internet) with the computer of the on-line patient. These changes are described in this document in a section that describes how the system uses networks, such as the internet, to perform its functions, and in a section that describes the impact of having a computer available at the patient's location.

The presently described system and method can be used via a remote login on a subject's computer or smartphone. The internet can serve as an access mechanism for running programs on a remote host computer. All of the application data and processes are stored and manipulated on the host computer. The internet serves only to transmit the user keystrokes and mouse clicks (or touchscreen entries) to the host, and to return the host computer output to the user computer or device. Remote login represents a way for the present system and method to use the internet as an I/O device for patients, doctors, dentists, laboratories, HMOs, and so on to gain access to the central computer. That is, patients log in to and are assessed by software running on the computer.

To engage in a two-way conversation between the remote user and the present system including the host computer, the user's response must be handled. This is done by internet software using a special protocol called CGI (Common Gateway Interface). Using CGI, when the user clicks a hypertext field in an HTML file, or fills in a HTML "form", the internet software sends a "reply" back to the original sender, where it is intercepted and handled by a CGI program. The CGI concept is to insert a software step that "catches" user responses and processes them. The CGI protocol supports two-way communication between the remote computers via the internet, and supports the storing of session-specific data between sessions. This preserves the "state" of the communication, so that a continuous dialog can be generated.

The features and advantages that CGI-invoked programs offer to the present system and method are that they: run on the central the computer, are part of the assessment / evaluation program library, can access central the present system and method databases, can accept and process patient inputs from the current HTML file, can calculate values based on patient data and inputs, can interpret mouse clicks on active screen images, so that the patient can respond by clicking on anatomical and medical charts, drawings, and photos, can appear to react to patient responses much like a Graphic User Interface (GUI)-based program, can be maintained and updated by the present system and method staff as needed.

The Java concept is an example of a software mechanism for attaching executable processes to the HTML page, transmitting them, and then running them on the receiving computer. Whereas CGI-invoked programs run on the sender's computer and are "blind" to the capabilities of the remote receiver's machine, the Java approach essentially permits both data and programs to be transmitted across the internet. The HTML programmer basically writes application source code in Java and appends it to the HTML page. When the user's browser displays the HTML page on the user's computer, it executes (actually "interprets") the Java code and handles the input/output to the user. Since the browser is written for the user's computer, it "knows" the user's system, and can take full advantage of this fact. The Java concept is merely an illustration of the concept of transmitting programs to the receiver. In fact, any executable software can be appended to the HTML page, provided the receiver knows how to read and interpret or execute it. Any number of other codes and languages can be used to do this for the present system and method. Ultimately, the present system and method can transmit the necessary software to its subscribers.

### Advantages of the present system and method on a network:

Using the internet and software mechanisms such as HTML, CGI, and Java permits the present system and method to: support internet communication protocols and formats; store charts, tables, graphs, images, photos, video, audio files; store internet pages and scripts (HTML, CGI, Java); stage scripts and other files for transmission; transmit pages and scripts as requested via the internet; upload medical and dental data collected by the present system and method and scripts from patients; download medical and dental data to patients, physicians, dentists, labs, and HMOs; download the scripts, programs, and data to patient computers; monitor and manage patient traffic, demand, and queuing; transmit medical and dental data in color, graphics, and sound formats; transmit executable code to user computers; use the users' Graphic User Interface (screen, mouse, dialogs); distribute its computational load to user computers; extend its assessment tools to include visual analysis; access other sites via the internet (such as HMOs, insurance carriers, credit agency, bank, attending and referred physicians, hospitals, clinics, recovery homes, laboratories, pharmacies, health supply stores; nurses, health practitioners, aides, emergency rooms, paramedics, ambulance services).

### Network Configuration

The user/patient communicates with a computing environment. The computing environment may include a single computer utilizing software or the computing environment may include multiple computers in a client/server relationship on a computer network. In a client/server environment, the server includes the presently described system which communicates with a client that may include a network terminal equipped with a video display, keyboard and pointing device. The network terminal is connected to a wide area network via a network connection, which may be either a dial-up connection using a modem and the public switched telephone network (PSTN) or via a dedicated data circuit. The wide area network can be a public network, like the internet, or a closed, private data network, like a corporate network or an intranet. There is an array of servers which host the medical and dental advice and treatment applications and the patient databases at a central location. These servers are connected via a local area network to a network gateway, which provides access to the wide area network via a high-speed, dedicated data circuit. Alternatively, a single server may host the medical and dental advice and treatment applications and the patient databases.

The patient's computer can be used to: download HTML pages from the presently disclosed system and method computer; execute program code (e.g. Java) embedded in the HTML; respond to questions posed on the page; upload responses via the internet to the computer; store system-related scripts, programs and utilities; print patient-oriented materials and reports; store the system-generated data; store patient medical and dental data; run scripts locally, off-line; save session data to continue later (and/or modify or re-enter); and offload or distribute the processing load from the presently described system and method's computer.

A computer handles graphic input/output at the patient's end, and the presently described system encodes medical script operations into some internet-compatible protocol, such as HTML. The software responsible for input/output may be swapped out for a module that handles the internet protocol. In addition to redirecting the input/output traffic, given the flexibility of a GUI-based operating system, the system can be enhanced in numerous ways. For example, using a GUI, the present system and method assessment scripts can: use various fonts and colors to highlight and emphasize output text; display graphic drawings and images to illustrate anatomical features; instruct, illustrate, and exemplify meanings of words and phrases; display photographic images as samples of lesions; display medical charts to compare the patient's health status to population averages; use multiple, overlapping or tiled screen displays to present data; display moving images to inquire about motion ranges; input one-digit or one-letter answers by pressing a key on the keyboard; present graphs, sketches, diagrams, images, photos, videos; present color, sound, and audio; input responses by way of edit boxes, checkboxes, and buttons; let the patient select and refine responses with mouse, joystick, keyboard; let the patient identify problems by selecting from pictures presented by the present system and method; enter textual data into fields displayed on the screen by the present system and method; enter lengthy textual descriptions using the keyboard; use labeled fields to let the patient "fill in a form" to be submitted to the present system and method; select responses from a list of choices presented on the screen; point to a selected area of an anatomical drawing or image; click the mouse to indicate intensities on a chart that shows a range of intensities; use hypertext and hypergraphics "links" to control the assessment sequence; provide various levels of "help" text tied to specific parts of the screen display.

The networked the present system and method system can include a network "cloud", which may represent a local area network (LAN), a wide area network (WAN), the internet, or another connection service. The programs and databases preferably reside on a group of servers that are preferably interconnected by a LAN and a gateway to the network. Alternatively, the programs and databases reside on a single server that utilizes network interface hardware and software. The servers store the assessment scripts used by the script engine.

The network may connect to a user computer, for example, by use of a modem or by use of a network interface card. The user at computer may utilize a browser to remotely access the programs using a keyboard and/or pointing device and a visual display, such as monitor. Alternatively, the browser is not used when the programs are executed in a local mode on computer. A still photograph or video camera may be optionally connected to the computer to provide visual input, such as visual symptoms.

Additional data entry points can be included and provided by electronic wearables and monitors (e.g., overnight monitor(s) for sleep or arrhythmia, wearables such as Fitbit^{®}, Apple^{™} "i-watch," Jawbone^{®} UP Fitness trackers and The Grommet, HRV - showing heart rhythm patterns, and Eccrine Systems' disposable sweat-monitoring device). Sweat testing can provide insights into the status of a human body and then transmit wirelessly into the cloud; its sensor could alert physicians when a subject stops taking medications or can tell the user whether the current workout is anaerobic or aerobic, and whether to back-off and slow down, or to pick up the pace. Other devices and derivatives of any of these data sources are encompassed by the present disclosure.

Various other devices may be used to communicate with the servers. If the servers are equipped with voice recognition or DTMF hardware, the user can communicate with the program by use of a telephone, as described in the telephonic embodiment above. Other connection devices for communicating with the servers include a portable personal computer with a modem or wireless connection interface, a cable interface device connected to a visual display, or a satellite dish connected to a satellite receiver and a television. Other ways of allowing communication between the user and the server are envisioned.

The presently preferred user/patient computer has several possible interconnections to the network. A program called a Script Engine may be used to "play" a script, which reads a medical and dental assessment scripts (MDAS) file and uses its codes to perform interview actions, such as outputting a question to a patient and inputting an answer. In some embodiments, the main memory in the system uses a disease/symptom/question (DSQ) list script engine. The script engine can collect answers from the patient, evaluate the answers, issue an assessment and/or prediction of prognosis, and update the patient's medical and dental records. The script engine can reside in the user computer. The script engine may be stored on the hard drive or CD-ROM, and is loaded into the main memory for execution. Other devices for conducting a medical and/or dental interview may be used in place of the computer.

As the user proceeds through an assessment process, information on the user's medical and dental condition is communicated to the presently described system by completing or answering screen-displayed forms or by pointing at an item on the display, using a touchscreen or clicking with a mouse. The assessment process retrieves data from the patient and other the system databases and stores the user's responses via CGI script language utilities or by use of a Web server application programming interface (API).

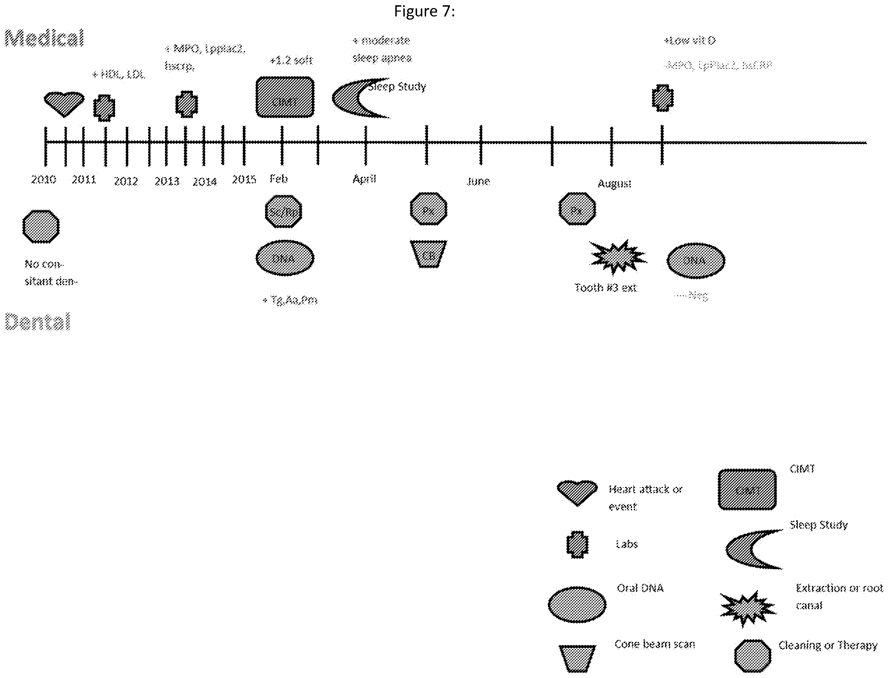

The medical and dental status and history are also assessed and related over time. Figure 7 shows an example of a time line representing a subject's medical and dental history and the predicted decrease in problematic events/results after implementing a treatment plan. In this example of a timeline, medical history and test results are shown above the line, and dental history and assessment are shown below the line. The subject's medical and dental history can include certain time frames to represent, for example, results of past annual physical exams. The time line can also include, but is not limited to, events such as previous heart attack(s), root canal(s), family history of diabetes, obesity / overweight status, dry mouth, frequent urination, insulin resistance, and other specific medical and dental events/details over time. Referring to Figure 7, "+" or "pos" is used to indicate positive test results (e.g., "+ Aa, Pm" indicates that bacterial pathogens *Aggregatibacter actinomycetemcomitans and Peptostreptococcus (Micromonas) micros* were detected. Thus, the subject's historic path of disease is known, and risks of future events can be predicted, and a treatment plan can be devised in conjunction with the other tools of the presently described method and system. With proper treatment and care prescribed using the present method and system, the number of events and levels or presence of pathogens is predicted to decrease over time, as the patient progresses on the prescribed path to Global Wellness. Such a timeline could even be incorporated into the subject-facing computer or wearable device as a way to show the patient's progress and to motivate continued implementation of the prescribed pathway.

If the user's network terminal is equipped with a camera, the system is capable of capturing imagery of the patient over time. For medical and or dental conditions that require treatment, a chronological sequence of images can allows the health care professional to make an assessment of the patient's condition by analyzing visual manifestations of an infection or disease.

Likewise, if the user's network terminal is equipped with a microphone, the system can capture clinical sounds provided by the patient (e.g., a cough or the wheezing of asthma). The present system can assist a health care professional in the assessment of a patient's medical and dental condition by playing back the clinical sounds it captured.

The presently described system may use several principal processes and related databases. A set of processes are used to register a patient or assistant. Similarly, a set of patient login processes can be used by the system to identify a patient who has previously registered into the system in various ways, such as: 1) by prompting for a patient identification number (PIN); 2) identifying an assistant who has previously registered into the system by prompting for an assistant identification number (AIN); 3) identifying a patient, having an assistant, who has previously registered into the system by prompting for the patient identification number.

Once a user has logged in or registered, the system provides a choice of processes. The primary process of concern in the current embodiment is the assessment process that performs a patient assessment. The evaluation process accesses a laboratory test of choice and imaging modality of choice database to recommend the appropriate tests in this patient at this point in time and a treatment table to obtain current treatment information for a particular disease or diagnosis. In another embodiment, other choices are added to access other medical and dental information processes.

Associated with these processes are a patient and assistant enrollment database, a consultation history database, a patient response database, a medical and dental history objects database, a patient medication database, a pending database, a patient medical history database, a medical and dental assessment scripts (MDAS) database, an imaging modality database, and a laboratory test database.

### Script Generation

An off-line process for generating a DSQ script starts with a process in which medical and dental knowledge is collected and organized into list files. The data for the list files is collected from one or more medical and dental authors. A first portion of the process is typically performed by a script coordinator or supervising author for assigning diseases and a second portion captures the disease knowledge for each disease in the script. The portion for capturing the disease knowledge is typically performed by a plurality of medical and dental experts in their respective fields. The output of process is electronic text, such as an ASCII file. This electronic text is in the form of DSQ lists such as disease, symptom, and question lists.

The process moves to state which takes the DSQ lists in electronic text format and processes them by use of a script data development tool. A script compiler works closely with the script data development tool to generate an MDAS file. The process may utilize the script data development tool and the script compiler in an iterative fashion to generate a final MDAS file. At state, the MDAS file is written to an MDAS database by an MDAS database manager utility. The MDAS file is preferably in a binary format. The MDAS preferably includes a header data section, a master disease list section, a master symptom list section, a master flows section, a master question list section and a master text list section. In another embodiment, the medical and dental authors may write the scripts in a medical and dental authoring language or as nodes and branches at state. Other script tools, which may include compilers, are at state to generate an MDAS.

### Run-Time Structures

Some of the files and components are used at run-time. The present system and method includes databases and files other than the MDAS database. If the script engine executes the script at the patient/user access processor, the network-based system passes the script to the gateway process that is part of the presently described system's network server, for transfer to the user processor. The gateway process is also known as a CGI program. Alternatively, a script engine at the presently disclosed system may utilize the script to provide questions to the user, respond to user input, and generate results across the network.

The patient/user access processor may utilize, in one embodiment, a Java virtual machine, which may or may not execute within the context of a browser. Another embodiment may utilize a plug-in script engine for the browser. The user access processor may be a Network PC (NetPC) such as defined by a collaboration of Intel, Microsoft, Compaq, Dell and Hewlett-Packard, or a Net Computer (NC), such as available from Oracle Corporation or Sun Microsystems Inc. The input devices used in conjunction with computer may be also with the user access processor.

The present system and method may interact on-line with the patient's computer via the network. One variant of the process is the ability for some pages (e.g., Java-enabled) to perform some calculations on the patient computer, instead of having to send the page to the computer. Beginning at a start state, process moves to state wherein the patient's computer contacts the central computer. Proceeding to state, the central the computer electronically sends a "page" or screen of electronic information to the patient computer for display on the visual display to the user. Continuing at state, the patient fills in form fields, check boxes, buttons, or other similar response mechanisms that are on the page. Advancing to state, the patient's computer sends the responses on the page back to the computer. Moving to state, the computer analyzes the received responses and selects the next page to send to the patient computer or determines, at state, if the user or the software desires to terminate the communication between the patient computer and the computer. If so, the process completes at an end state. However, if process determines not to terminate, execution continues back at state wherein the system sends the next selected page to the patient's computer.

## Claims

1. A system for identifying an oral-systemic link and improving global systemic wellness in a subject, said system comprising:
(i) a computer-readable storage medium comprising recorded medical and dental history data points from the subject;
(ii) a sample-collection and measuring apparatus for obtaining an oral sample from the subject, comprising a plurality of analyte detection regions in direct or indirect fluid communication with said sample-collection and measuring apparatus, wherein the analyte detection regions test for the presence of and measure current levels of:
(a) proteins and nucleic acids from bacteria, viruses or other pathogens; and
(b) endogenous genetic biomarkers of inflammation, infection, and autoimmune disease; and/or
(c) specific hormones or lipids;
(iii) results from a current periodontal analysis;
(iv) a computer program that compares the medical and dental history data points of (i), the current levels from the analyte detection regions of (ii), and the results from the current periodontal analysis of (iii) to a reference set of desired health and wellness goals, identifies oral-systemic links, generates an initial digital wellness landscape and a restored wellness landscape, and calculates, *in silico*, a plan comprising a series of behavioral steps according to a minimum step criterion, such that, when the behavioral steps are performed by the subject, the subject is treated and improves in global systemic wellness.

## Patentansprüche

1. System zum Identifizieren einer oral-systemischen Verbindung und zum Verbessern der globalen systemischen Wellness bei einem Subjekt, wobei das System Folgendes umfasst:
(i) ein computerlesbares Speichermedium, das aufgezeichnete Datenpunkte der medizinischen und zahnmedizinischen Vorgeschichte von dem Subjekt umfasst;
(ii) eine Probenentnahme- und Messvorrichtung zum Gewinnen einer oralen Probe von dem Subjekt, umfassend eine Vielzahl von Analytdetektionsbereichen, die in direkter oder indirekter Fluidkommunikation mit der Probenentnahme- und Messvorrichtung stehen, wobei die Analytdetektionsbereiche auf das Vorhandensein von Folgenden testen und deren aktuelle Spiegel messen:
(a) Proteine und Nukleinsäuren von Bakterien, Viren oder anderen Erregern; und
(b) endogene genetische Biomarker für Entzündung, Infektion und Autoimmunerkrankung; und/oder
(c) spezifische Hormone oder Lipide;
(iii) Ergebnisse einer aktuellen Parodontalanalyse;
(iv) ein Computerprogramm, das die Datenpunkte der medizinischen und zahnmedizinischen Vorgeschichte von (i), die aktuellen Spiegel aus den Analytdetektionsbereichen von (ii) und die Ergebnisse aus der aktuellen Parodontalanalyse von (iii) mit einem Referenzsatz von erwünschten Gesundheits- und Wellnesszielen vergleicht, oral-systemische Verknüpfungen identifiziert, eine anfängliche digitale Wellnesslandschaft und eine Landschaft der wiederhergestellten Wellness erzeugt und *in silico* einen Plan berechnet, der eine Reihe von Verhaltensschritten nach einem Mindestschrittkriterium umfasst, sodass das Subjekt behandelt wird und sich seine globale systemische Wellness verbessert, wenn die Verhaltensschritte von dem Subjekt durchgeführt werden.

## Revendications

1. Système d'identification d'un lien oral-systémique et d'amélioration du bien-être systémique global chez un sujet, ledit système comprenant :
(i) un support de stockage lisible par ordinateur comprenant des points de données d'historique médical et dentaire enregistrés du sujet ;
(ii) un appareil de collecte et de mesure d'échantillons pour obtenir un échantillon oral du sujet, comprenant une pluralité de régions de détection d'analytes en communication fluidique directe ou indirecte avec ledit appareil de collecte et de mesure d'échantillons, dans lequel les régions de détection d'analytes testent la présence et mesurent les niveaux actuels :
(a) de protéines et d'acides nucléiques provenant de bactéries, de virus ou d'autres agents pathogènes ; et
(b) de biomarqueurs génétiques endogènes d'une inflammation, d'une infection et de maladies auto-immunes ; et/ou
(c) d'hormones ou des lipides spécifiques ;
(iii) les résultats d'une analyse parodontale actuelle ;
(iv) un programme informatique qui compare les points de données d'historique médical et dentaire (i), des niveaux actuels des régions de détection d'analytes de (ii), et des résultats de l'analyse parodontale actuelle de (iii) à un ensemble de référence d'objectifs de santé et de bien-être souhaités, identifie les liens oraux-systémiques, génère un bien-être général numérique initial et un bien-être général restauré, et calcule, *in silico*, un plan comprenant une série d'étapes comportementales selon un critère d'étape minimum, de sorte que, lorsque les étapes comportementales sont exécutées par le sujet, le sujet est traité et s'améliore en termes de bien-être systémique global.
